(19) Europäisches Patentamt
European Patent Office
Office européen des brevets

(11) EP 0 373 988 B2

(12) NOUVEAU FASCICULE DE BREVET EUROPEEN

(45) Date de publication et mention de la décision concernant l'opposition:
**17.10.2001 Bulletin 2001/42**

(51) Int Cl.[7]: **A61K 7/00**, A61K 7/06, A61K 9/127

(45) Mention de la délivrance du brevet:
**11.11.1992 Bulletin 1992/46**

(21) Numéro de dépôt: **89403225.9**

(22) Date de dépôt: **22.11.1989**

(54) **Composition cosmétique ou pharmaceutique pour le traitement des cheveux et du cuir chevelu**

Kosmetische oder pharmazeutische Zubereitung zur Haar- und Haarbodenbehandlung

Cosmetic or pharmaceutical composition for hair and scalp treatment

(84) Etats contractants désignés:
**AT BE CH DE ES FR GB GR IT LI NL SE**

(30) Priorité: **02.12.1988 LU 87399**

(43) Date de publication de la demande:
**20.06.1990 Bulletin 1990/25**

(73) Titulaire: **L'OREAL**
**75008 Paris (FR)**

(72) Inventeurs:
- **Grollier, Jean-François**
**F-75004 Paris (FR)**
- **Richoux, Isabelle**
**F-75020 Paris (FR)**

(74) Mandataire: **Peuscet, Jacques**
**SCP Cabinet Peuscet et Autres,**
**78, avenue Raymond Poincaré**
**75116 Paris (FR)**

(56) Documents cités:
**EP-A- 0 138 395**
**WO-A-88/06881**
**DE-A- 2 619 100**
**GB-A- 2 157 168**
**JP-A- 60 019 710**
**JP-A- 63 063 606**
**JP-A- 63 230 620**
**US-A- 4 772 471**



Printed by Jouve, 75001 PARIS (FR)

## Description

La présente invention concerne une composition cosmétique ou pharmaceutique pour le traitement des cheveux et du cuir chevelu.

Il est bien connu que les cheveux sont sensibilisés ou fragilisés à des degrés divers par l'action des agents atmosphériques ainsi que par l'action de différents traitements cosmétiques, tels que permanentes, teintures ou décolorations. Les cheveux deviennent alors difficiles à démêler et à coiffer. De plus, ils deviennent rêches au toucher.

On a donc cherché des compositions, qui permettent de faciliter le démêlage et le coiffage des cheveux et améliorent leur douceur au toucher. Dans ce but, on utilise couramment des agents tensio-actifs cationiques. Lesdits agents tensio-actifs améliorent le démêlage et le coiffage mais présentent des inconvénients : ils ont tendance à alourdir la chevelure et à lui donner un aspect gras. Ces inconvénients sont d'autant plus accentués que les cheveux traités sont plus fins.

Dans le même but, on a également proposé d'utiliser des protéines quaternisées. Lesdites protéines ne présentent ni l'inconvénient d'alourdir les cheveux, ni celui de leur donner un aspect gras ; par contre, l'amélioration du démêlage et du coiffage des cheveux, qu'elles apportent, est nettement inférieure à celle apportée par les agents tensio-actifs cationiques.

On a donc essayé d'utiliser des compositions contenant à la fois un agent tensio-actif cationique et une protéine quaternisée. Mais, dans ce cas, l'effet obtenu est inférieur à la somme des effets que l'on pourrait obtenir séparément à l'aide de l'agent tensio-actif cationique et à l'aide de la protéine quaternisée car, dans la majorité des cas, le dépôt de l'agent tensio-actif cationique sur le cheveu freine celui de la protéine quaternisée. On a déjà proposé, dans GB-A-2 157 168, de remédier à cette situation par addition d'un polymère silicone cationique, qui améliore de façon sensible l'effet obtenu sur le cheveu.

Par ailleurs, il est connu, de très longue date, d'utiliser des huiles et des corps gras pour redonner aux cheveux de la douceur et du brillant ; l'application de ces composés est généralement suivie d'un shampooing pour éliminer du cheveu l'excédent d'huile ou de corps gras. Cependant, l'utilisation des huiles et corps gras amollit et alourdit les cheveux et il est impossible d'obtenir par la suite une coiffure ayant de la tenue et du volume.

Selon la présente invention, on a trouvé qu'en associant, à une solution aqueuse contenant au moins un agent tensio-actif cationique et/ou une protéine quaternisée, des lipides non ioniques susceptibles de former une phase lamellaire lipidique hydratée insoluble dans l'eau, on obtenait des compositions, qui permettent de démêler et coiffer facilement les cheveux sans les ramollir, ni les alourdir, ni les graisser. De plus, lesdites compositions confèrent aux cheveux des propriétés surprenantes quant au gonflant et permettent un gainage des cheveux de la racine à la pointe. Les cheveux traités ont donc du brillant et de la douceur et, après démêlage, ils sont lisses et légers de la racine à la pointe, même dans le cas des cheveux sensibilisés et des cheveux fins. Ces effets sont comparables à ceux définis par GB-A-2 157 168.

Mais on a également trouvé que les compositions selon la présente invention présentent, de façon surprenante, un effet hydratant sur le cuir chevelu. Elles ont l'avantage de procurer une sensation agréable de fraîcheur et de confort sur le cuir chevelu. Elles ont donc une double action : sur les cheveux et sur le cuir chevelu ; ce résultat est tout à fait nouveau et inattendu.

De plus, les compositions selon l'invention ont une excellente stabilité au stockage.

La présente invention a donc pour objet une composition cosmétique ou pharmaceutique pour le traitement des cheveux et du cuir chevelu renfermant des lipides amphiphiles non-ioniques susceptibles de former une phase lamellaire lipidique hydratée, insoluble dans l'eau, éventuellement associés à un stabilisant, lesdits lipides étant dispersés dans une phase aqueuse continue, caractérisée par le fait que ladite phase aqueuse contient:

1) au moins un agent tensio-actif cationique de formule :

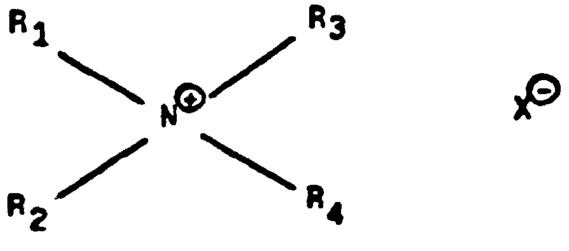

(I)

formule dans laquelle X est notamment le chlore ou $CH_3\ SO^-_4$ et $R_1$ est un radical alkyle en $C_1$ - $C_4$, de préférence

le radical méthyle, et dans laquelle :

a) lorsque X est le chlore :

- ou bien $R_2$ et $R_3$ sont des radicaux alkyle en $C_1$ - $C_4$, identiques ou différents de $R_1$ et entre eux, et $R_4$ est un radical alkyle en $C_{16}$ - $C_{22}$;
- ou bien $R_2$ = $R_1$ et, dans ce cas :
  - ou $R_3$ = $R_4$ = radical alkyle en $C_{18}$;
  - ou $R_3$ = radical (alkyle $C_{17}$)amidopropyle et $R_4$ = radical (alkyle $C_{14}$)acétate ;

b) lorsque X est $CH_3SO^-_4$:

- $R_2$ désigne un radical (alkyle et/ou alkényl)amidoéthyle, dans lequel le radical alkyle et/ou alkényle est en $C_{13}$ - $C_{21}$ et dérive des acides gras du suif ;
- $R_3$ et $R_4$ forment ensemble avec l'azote un hétérocycle 4-5-dihydroimidazole substitué, notamment un radical 2-(alkyle $C_{13}$ - $C_{21}$ dérivé des acides gras du suif)4,5-dihydroimidazole

et/ou
2) au moins une protéine quaternisée constituée par un polypeptide modifié chimiquement portant, en bout de chaîne ou greffé sur celle-ci, au moins un groupement ammonium quaternaire qui contient au moins un groupe alkyle en $C_1$ - $C_{18}$, le polypeptide étant choisi parmi les hydrolysats de protéine animale, le (s) agent(s) tensio-actif(s) représentant de 0,05 à 10 % en poids par rapport au poids total de la composition, la (les) protéine (s) quaternisée (s) représentant de 0,05 à 3 % en poids par rapport au poids total de la composition, le (les) lipide(s) amphiphile (s) non ionique (s) représentant de 0,1 à 20 % en poids par rapport au poids total de la composition.

Lorsque cette phase lamellaire lipidique hydratée forme des vésicules, celles-ci sont appelées des niosomes. Les niosomes contenus dans la composition selon l'invention ont avantageusement un diamètre moyen compris entre 0,01 et 5 µm de préférence entre 0,1 et 0,35 µm.

Les niosomes sont connus dans l'état de la technique. Ils sont décrits, ainsi que leur procédé de préparation, dans FR-A-2 315 991, US-A-4 772 471 et WO-88/06881. Ce sont des sphérules ou vésicules constituées d'une ou plusieurs couches concentriques de lipides séparées par des couches de phase aqueuse interne. Dans le cas des niosomes, les lipides utilisées pour la fabrication des sphérules ou vésicules sont, de façon connue, des amphiphiles non-ioniques, d'origine naturelle ou synthétique, comportant, par molécule, une ou plusieurs longue (s) chaîne(s) hydrocarbonée(s).

Selon l'invention, le(s) composé(s) lipidique(s) amphiphile(s) non-ionique(s) constituant la phase lamellaire lipidique hydratée, insoluble dans l'eau, sont avantageusement choisis parmi les éthers ou esters de polyglycérol, linéaires ou ramifiés, de formules respectives :

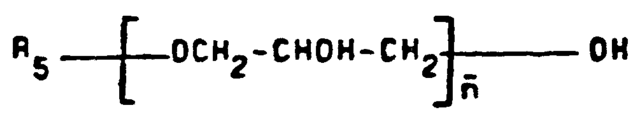

(II)

et

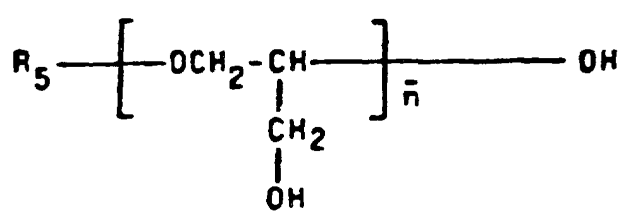

(III)

formules où $\bar{n}$ est une valeur statistique moyenne comprise entre 2 et 6 et où $R_5$ est :

1) ou bien une chaîne aliphatique $R_6$ ou un reste $R'_6$ CO, $R_6$ étant un radical aliphatique, linéaire ou ramifié, en $C_{12}$- $C_{18}$ et $R'_6$ un radical aliphatique, linéaire ou ramifié, en $C_{11}$ - $C_{17}$;
2) ou bien un radical

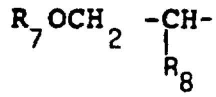 ,

où $R_7$ et $R_8$ sont des radicaux $R_6$ ou $R'_6$ CO, identiques ou différents, $R_6$ et $R'_6$ ayant les significations ci-dessus définies.

De façon connue, ces composés lipidiques non-ioniques constituant la phase lamellaire lipidique hydratée sont, de préférence, associés à au moins un additif stabilisant destiné à modifier la perméabilité ou la charge superficielle des feuillets lipidiques de la phase lamellaire lipidique hydratée. Selon l'invention, ces additifs sont plus particulièrement choisis dans le groupe formé par les stérols, tels que le cholestérol ou le bêta-sitostérol ; les sels monosodiques ou disodiques de glutamates d'acyle, le radical acyle étant en $C_{14}$-$C_{22}$, tel que le sel monosodique du glutamate de stéaroyle, les sels disodiques de glutamates de cocoyle, de stéaroyle ou de mélanges de radicaux acylés dérivés du coprah et du suif ; et des esters phosphoriques d'alcools gras en $C_{12}$- $C_{16}$.
Dans le cas où le lipide amphiphile non-ionique est un composé de formule (II) ou (III) définie ci-dessus, où $R_5$ représente $R_6$ ou $R'_6$ CO, $R_6$ et $R'_6$ ayant les significations ci-dessus indiquées, ledit lipide amphiphile non-ionique est associé, à la fois, à un stérol, de préférence le cholestérol et à un additif anionique.
Les stabilisants anioniques sont associés aux composés lipidiques amphiphiles non-ioniques en une quantité ne dépassant, de préférence, pas 12 % en poids par rapport au poids du (ou des) lipide(s) amphiphile(s) non-ionique(s) constituant la phase lamellaire lipidique hydratée. Pour les stérols, et notamment le cholestérol, cette même proportion doit rester inférieure ou égale à 100 % en poids.
Selon l'invention, la phase lamellaire lipidique hydratée contient de l'eau en présence ou non d'un agent cosmétiquement ou pharmaceutiquement actif. A titre d'agent actif, on peut citer, par exemple, les agents anti-chute ou de repousse des cheveux, les rétinoïdes et apparentés, les anti-inflammatoires, les anti-fongiques, les anti-séborrhéiques, les filtres solaires, et analogues.
L'agent tensio-actif cationique contenu, selon l'invention, dans la phase aqueuse continue de la composition, est avantageusement choisi dans le groupe formé par :

a) les halogénures de tétraalkylammonium tels que le chlorure de béhényltriméthylammonium, le chlorure de distéaryldiméthylammonium, le chlorure de triméthylcétylammonium ;
b) le chlorure de stéaramidopropyldiméthyl(myristyl acétate)ammonium de formule :

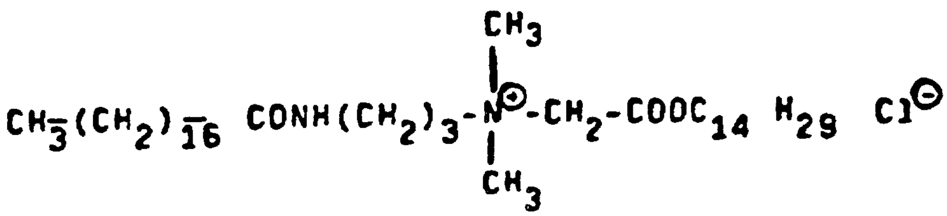 (IV)

tel que le produit vendu sous la dénomination commerciale "CERAPHYL 70" par la Société "VAN DYK";
c) un sel d'ammonium quaternaire de formule :

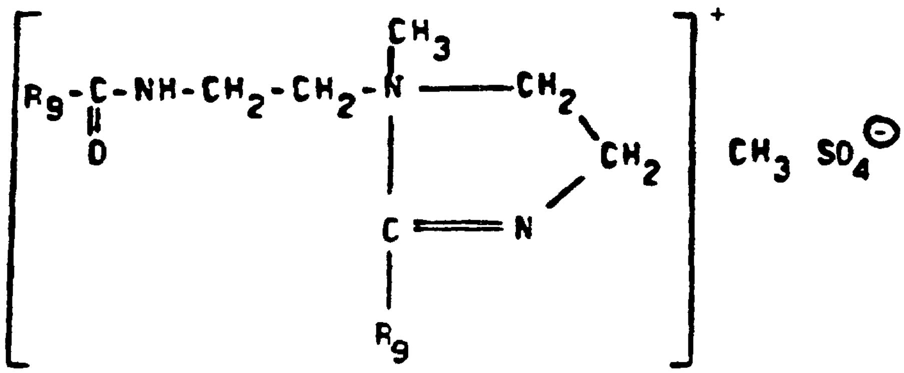

(V)

dans lequel $R_9$ désigne un mélange de radicaux alkényle et/ou alkyle en $C_{13}$ - $C_{21}$ dérivé des acides gras du suif, tel que le produit vendu sous la dénomination commerciale "REWOQUATW 7500" par la Société "REWO".

La protéine quaternisée contenue selon l'invention dans la phase aqueuse continue est avantageusement choisie dans le groupe formé par :

a) certains hydrolysats de protéine porteurs, sur la chaîne polypeptidique, de groupements ammonium quaternaires comportant au moins un radical alkyle en $C_1$ - $C_{18}$ et notamment un hydrolysat de protéine animale vendu sous la dénomination commerciale "CROTEIN Q" par la Société "CRODA", dont la chaîne polypeptidique a un poids moléculaire moyen de l'ordre de 12000 ;
b) des hydrolysats de protéine animale portant des groupements triméthylbenzylammonium (dénommés "benzyltrimonium hydrolysed animal protein" dans le "CTFA cosmetic Dictionary" (3ème édition, 1982) publié par "The Cosmetic Toiletry and Fragance Association Inc." et ci-après appelé "dictionnaire CTFA") et, par exemple, celui vendu sous la dénomination commerciale "CROTEIN BTA" par la Société "CRODA";
c) des hydrolysats de collagène porteurs de groupements triéthylammonium, dénommés "Triéthonium hydrolysed collagen ethosulfate" dans le dictionnaire CTFA et vendus sous la dénomination commerciale de "QUAT-PRO E" par la Société "MAYBROOK";
d) des hydrolysats de collagène porteurs de groupements triméthylammonium et triméthylstéarylammonium, dénommés dans le dictionnaire CTFA "Steartrimonium hydrolysed collagen" et vendus sous la dénomination commerciale "QUAT PRO S" par la Société "MAYBROOK";
e) une protéine quaternisée résultant de la condensation de la cocamidopropyl-diméthyl-amine sur une protéine animale hydrolysée, dénommée, dans le supplément de la 3e édition (1982) du dictionnaire CTFA : COCAMIDOPROPYLDIMONIUM HYDROXYPROPYLAMINO HYDROLYSED ANIMAL PROTEIN vendue sous la dénomination commerciale "LEXEIN QX 3000" par la Société "INOLEX".

Les compositions selon l'invention peuvent, de préférence, contenir, à la fois, au moins un tensio-actif cationique et au moins une protéine quaternisée. Dans ce cas :

- l'agent tensio-actif cationique est, de préférence, un chlorure de tétraalkylammonium de formule (I) dans lequel $R_1$, $R_2$ et $R_3$ sont des radicaux alkyle en $C_1$ - $C_4$ et $R_4$ est un radical alkyle en $C_{20}$ - $C_{22}$;
- et la protéine quaternisée est, de préférence, un hydrolysat de protéine animale porteur de groupements ammonium quaternaires comportant un radical alkyle en $C_1$ - $C_{18}$.

La phase aqueuse continue de dispersion peut éventuellement contenir, en plus de l'agent tensio-actif cationique et/ou de la protéine quaternisée, au moins un additif connu tel que des conservateurs, des stabilisants, des colorants, des parfums, des adoucissants, des humectants choisis, de préférence, parmi les polyols comme la glycérine, et des épaississants, tels que des alcools gras oxyéthylénés ou non.
Dans la composition selon l'invention, le (ou les) agent(s) tensio-actif(s) cationique(s) représente(nt) de 0,05 à 10 % en poids, de préférence de 0,1 à 6 % en poids, par rapport au poids total de la composition ; la (ou les) protéine(s) quaternisée(s) représente(nt) de 0,05 à 3 % en poids, de préférence de 0,05 à 0,5 % en poids, par rapport au poids total de la composition ; le (ou les) lipide(s) amphiphile(s) non-ionique(s), qui constitue(nt) la phase lamellaire représente(nt) de 0,1 % à 20 % en poids, de préférence de 1 % à 10 % en poids, et plus particulièrement 3 à 10 % par rapport au poids total de la composition.
Il est bien entendu que, dans le cas où l'on utilise une grande quantité d'agent(s) tensio-actif(s) cationique(s)

par rapport au(x) lipide(s) amphiphile(s) non-ionique(s), le choix du (ou des) agent(s) cationique(s) n'est pas indifférent et doit être effectué de manière qu'il(s) ne détruise(nt) pas les vésicules.

La composition selon l'invention est généralement préparée par mélange de deux constituants (A) et (B). Le constituant (A) contient la phase lamellaire lipidique hydratée, dans une phase aqueuse continue. Le constituant (B) contient, en phase aqueuse, l'agent cationique et/ou la protéine quaternisée. Chacun des constituants peut renfermer, en outre, divers additifs. On préfère que chacun des constituants (A) et (B) représentent 40 à 60 % en poids par rapport au poids total de la composition ; avantageusement, les deux constituants mélangés ont sensiblement le même poids.

Le constituant (A) est préparé de façon classique et plus particulièrement selon le procédé décrit dans le brevet français n° 2 315 991 :

- dans une première étape, on met en contact avec de l'eau les lipides amphiphiles non-ioniques, éventuellement mélangés à des additifs destinés à modifier la perméabilité ou la charge des feuillets lipidiques de la phase lamellaire lipidique hydratée que l'on veut former ;
- dans une deuxième étape, on ajoute à la phase lamellaire lipidique hydratée ainsi obtenue une phase aqueuse de dispersion ;
- dans une troisième étape, on soumet le mélange à une agitation énergique pour obtenir des vésicules.

Une fois le constituant (B) préparé, on l'ajoute au constituant (A), sous agitation, jusqu'à complète homogénéisation.

Les compositions selon l'invention sont, de préférence appliquées sous forme de produits à rincer, avant et plus particulièrement après un shampooing, avant et plus particulièrement après une coloration ou décoloration, avant et plus particulièrement après permanente ou défrisage. Elles peuvent également être appliquées sous forme de produits non rincés, par exemple, avant la mise en plis ou le brushing.

Pour la mise en oeuvre des compositions selon l'invention en vue d'un traitement des cheveux et/ou du cuir chevelu, on applique sur le substrat à traiter une quantité efficace de la composition selon l'invention, on laisse en contact pendant 1 à 15 mn avant de rincer quand il s'ag it de produit à rincer. Les quantités de composition appropriées sont, en général, de l'ordre de 20 à 40 g pour une tête dans le cas des produits à rincer et de l'ordre de 5 à 10 g dans le cas des produits non rincés.

Les exemples donnés ci-dessous, à titre purement illustratif, permettront de mieux comprendre l'invention.

EXEMPLE 1

Dans une première étape, on prépare un constituant A comprenant les vésicules. On fond, en agitant doucement à une température de 80°C, un mélange de 6 g de lipide non-ionique de formule :

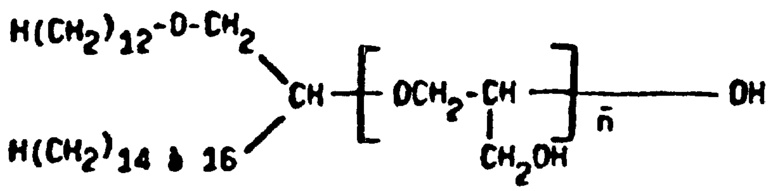

où $\bar{n}$ est une valeur statistique moyenne égale à 6, avec 1,6 g de cholestérol.

On introduit dans le mélange fondu 16 g d'eau portée à 90°C contenant un conservateur et l'on mélange pendant environ 5 mn. A la phase ainsi obtenue, on ajoute 24 g d'eau à 20°C ; on agite le mélange puis on ajoute encore 2,3 g d'eau à 20°C.

Dans une seconde étape, on prépare un constituant (B) par mélange des formulations B1 et B2 suivantes :

| Formulation B1 | |
|---|---|
| Alcool cétylique/alcool stéarylique (30/70) | 2,25 g |
| Alcool cétylique/alcool stéarylique oxyéthyléné à 33 moles d'oxyde d'éthylène | 0,55 g |
| Octyldodécanol | 0,4 g |
| Alcool cétylique/alcool stéarylique (50/50) | 1,2 g |

| Formulation B2 | |
|---|---|
| Glycérine | 0,4 g |
| Chlorure de béhényltriméthylammonium vendu sous la dénomination commerciale "GENAMIN KDM-F" par la Société "HOECHST" | 3,1 g |
| Hydrolysat de protéine, comportant une chaîne polypeptidique ayant un poids moléculaire d'environ 12000 et des groupements ammonium quaternaires porteurs d'au moins un groupe alkyle en $C_1$ - $C_{18}$, vendu sous la dénomination commerciale de "CROTEIN Q" par la Société "CRODA" | 0,25 g |
| Conservateur | q.s. |
| Eau | 40 g |

Le mélange des formulations B1 et B2 est effectué sous agitation et on maintient le mélange sous agitation jusqu'à complet refroidissement.

Dans une troisième étape, on réalise le mélange du constituant A et du constituant B et on le maintient sous agitation jusqu'à complète homogénéisation.

On applique cette composition à raison d'environ 25 g sur le cuir chevelu et les cheveux lavés et essorés. Après 10 mn de temps de pose, on rince à l'eau. On constate une sensation de fraîcheur à l'application sur le cuir chevelu. Après séchage, on voit que les cheveux sont gainés et que la coiffure est légère et gonflante. Les cheveux se démêlent aisément, sont brillants, doux et lisses jusqu'à leurs pointes.

EXEMPLE 2

On prépare une crème capillaire à rincer en procédant selon la technique décrite dans l'exemple 1. Le constituant (A) est préparé en agitant doucement à la température de 80°C un mélange de 3,8 g du lipide non-ionique de formule :

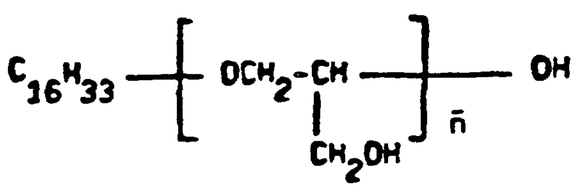

où $\bar{n}$ est une valeur statistique moyenne égale à 3, avec 3,8 g de cholestérol et 0,4 g du sel monosodique du glutamate de formule :

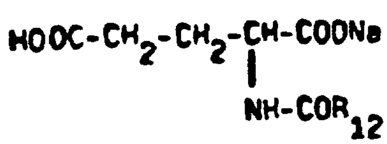

où $R_{12}$ est un mélange de radicaux alkényle et/ou alkyle hydrogénés en $C_{13}$ - $C_{21}$ dérivé des acides gras du suif, vendu sous la dénomination commerciale : "Acylglutamate HS 11" par la Société "AJINOMOTO".

A ce mélange fondu, on additionne ensuite de l'eau portée à 90°C renfermant un conservateur et l'on mélange pendant quelques minutes. Ala phase ainsi obtenue, on ajoute ensuite sous agitation de l'eau froide pour compléter à 50 g.

On introduit ensuite, dans le constituant (A) ainsi préparé, le constituant (B) décrit dans l'exemple 1 et on agite jusqu'à homogénéisation.

Cette crème est appliquée sur le cuir chevelu et les cheveux lavés et essorés ; elle a les mêmes avantages que celle obtenue dans l'exemple 1.

EXEMPLE 3

On opère comme dans l'exemple 1, en utilisant dans le constituant (B) la formulation B2 suivante :

| | |
|---|---|
| Glycérine | 0,4 g |
| Hydrolysat de collagène à groupements triéthylammonium vendu sous la dénomination commerciale "QUAT PRO E" par la Société "MAYBROOK" | 1 g |
| Conservateur | q.s. |
| Eau | 43 g |

Cette crème est appliquée sur le cuir chevelu et les cheveux lavés et essorés ; elle a les mêmes avantages que celle obtenue dans l'exemple 1.

EXEMPLE 4

On reproduit l'exemple 1 en utilisant dans le constituant (B) la formulation B2 suivante :

| | |
|---|---|
| Glycérine | 0,4 g |
| Chlorure de distéaryldiméthylammonium | 6 g |
| Conservateur | q.s. |
| Eau | 38 g |

Cette crème est appliquée sur le cuir chevelu et les cheveux lavés et essorés ; elle a les mêmes avantages que celle obtenue dans l'exemple 1.

EXEMPLE 5

On opère comme dans l'exemple 1 en utilisant dans le constituant (B) la formulation B2 suivante :

| | |
|---|---|
| Glycérine | 0,4 g |
| Sel d'ammonium quaternaire vendu sous la dénomination commerciale "REWOQUAT 7500 PG" par la Société "REWO" | 5 g |
| Conservateur : | q.s. |
| Eau | 40 g |

Cette crème est appliquée sur le cuir chevelu et les cheveux lavés et essorés ; elle a les mêmes avantages que celle obtenue dans l'exemple 1.

EXEMPLE 6

On prépare une crème capillaire à rincer en procédant selon la technique décrite dans l'exemple 1. Le constituant (A) est préparé en agitant doucement à la température de 80°C un mélange de 3,8 g du lipide amphiphile non-ionique de formule :

$$C_{15}H_{31}-CO-\left[OCH_2-CHOH-CH_2\right]_2-OH$$

avec 3,8 g de cholestérol et 0,4 g du sel monosodique du glutamate de formule :

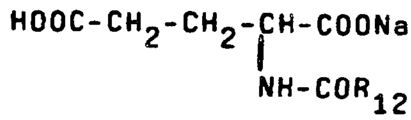

où $R_{12}$ est un mélange de radicaux alkényle et/ou alkyle hydrogénés en $C_{13}$ - $C_{21}$ dérivé des acides gras du suif, vendu sous la dénomination commerciale "Acylglutamate HS 11" par la Société "AJINOMOTO".

A ce mélange fondu, on ajoute ensuite de l'eau portée a 90°C renfermant un conservateur et l'on mélange pendant quelques minutes. A la phase ainsi obtenue, on ajoute ensuite sous agitation de l'eau froide pour compléter à 50 g.

On introduit ensuite, dans le constituant (A) ainsi préparé, le constituant (B) décrit dans l'exemple 1 et on agite jusqu'à homogénéisation.

Cette crème est appliquée sur le cuir chevelu et les cheveux sont lavés et essorés ; elle a les mêmes avantages que celle obtenue dans l'exemple 1.

EXEMPLE 7

On reproduit l'exemple 2 en remplaçant les 0,4 g du sel monosodique du glutamate de formule décrite dans ledit exemple 2 par 0,4 g de dihexadécylphosphate de sodium.

On obtient une crème, qui a les mêmes effets sur les cheveux que ceux de la crème obtenue dans l'exemple 2.

EXEMPLE 8

On prépare une crème antipelliculaire sur la base de la composition de l'exemple 1 en introduisant au mélange fondu de lipide et de cholestérol, 0,5 g du sel d'éthanolamine de l'hydroxy-1, méthyl-4 (triméthyl-2,4,4)-pentyl-61H-pyridone-2 vendu sous la dénomination commerciale "OCTOPIROX" par la Société "HOECHST".

Après des applications bihebdomadaires pendant 3 mois, on observe une régression du nombre des pellicules.

L'effet cosmétique sur le cheveu est le même que celui décrit dans l'exemple 1.

EXEMPLE 9

On prépare une crème capillaire sur la base de la composition de l'exemple 1, en introduisant au mélange fondu du lipide et du cholestérol, 0,5 g d'un dérivé terpénique (BISABOLOL) vendu sous la dénomination commerciale "DRAGOSANTOL" par la Société "DRAGOCO".

Par application de cette crème, en particulier après une permanente, un défrisage ou une décoloration, l'utilisateur ressent une sensation apaisante sur le cuir chevelu.

Après séchage, les cheveux sont gainés, la coiffure est légère et gonflante. Les cheveux se démêlent aisément, sont brillants, doux et lisses jusqu'à leurs pointes.

EXEMPLE 10

On prépare une crème capillaire sur la base de la composition de l'exemple 1, en introduisant dans 16 g d'eau portée à 90°C et contenant un conservateur, 3 g de l'agent hydratant vendu sous la dénomination commerciale "PRODEW 100" par la Société "AJINOMOTO".

Lors de l'application, on obtient un effet hydratant sur le cuir chevelu.

On retrouve les mêmes avantages sur le cheveu que ceux décrits dans l'exemple 1.

**Revendications**

**Revendications pour les Etats contractants suivants : AT, BE, CH, DE, FR, GB, IT, LI, NL, SE**

**1.** Composition cosmétique ou pharmaceutique pour le traitement des cheveux renfermant des lipides amphiphiles non-ioniques susceptibles de former une phase lamellaire lipidique hydratée insolubles dans l'eau, éventuellement

associés à un stabilisant, sous forme de vésicules dispersées dans une phase aqueuse continue, **caractérisée par le fait que** ladite phase aqueuse contient :

1) au moins un agent tensio-actif cationique de formule :

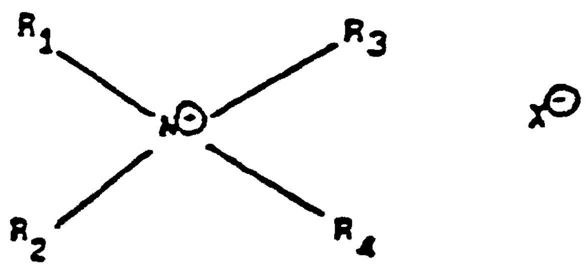

(I)

formule dans laquelle X est le chlore ou $CH_3SO^-_4$ et $R_1$ est un radical alkyle en $C_1$ - $C_4$ et dans laquelle :

a) lorsque X est le chlore :

- ou bien $R_2$ et $R_3$ sont des radicaux alkyle en $C_1$ - $C_4$, identiques ou différents de $R_1$ et entre eux, et $R_4$ est un radical alkyle en $C_{16}$ - $C_{22}$;
- ou bien $R_2$ = $R_1$ et, dans ce cas :

  soit $R_3$ = $R_4$ = radical alkyle en $C_{18}$;
  soit $R_3$ = radical (alkyl $C_{17}$)amidopropyle et $R_4$ = radical (alkyl $C_{14}$)acétate et

b) lorsque X est $CH_3SO^-_4$:

- $R_2$ désigne un radical (alkyl et/ou alkényl amidoéthyle, dans lequel le radical alkyle et/ou alkényle est un radical en $C_{13}$- $C_{21}$ et dérive des acides gras du suif;
- $R_3$ et $R_4$ forment ensemble avec l'azote un cycle 4,5-dihydroimidazole substitué en position 2; et/ou

2) au moins une protéine quaternisée constituée par un polypeptide modifié chimiquement portant, en bout de chaîne ou greffé sur celle-ci, au moins un groupement ammonium quaternaire qui contient au moins un groupe alkyle en $C_1$ - $C_{18}$, le polypeptide étant choisi parmi les hydrolysats de protéine animale, le (s) agent (s) tensio-actif(s) représentant de 0,05 à 10 % en poids par rapport au poids total de la composition, la (les) protéine (s) quaternisée (s) représentant de 0,05 à 3 % en poids par rapport au poids total de la composition, le (les) lipide (s) amphiphile (s) non ionique (s) représentant de 0.1 à 20 % en poids par rapport au poids total de la composition.

2. Composition selon la revendication 1, **caractérisée par le fait que** les vésicules ont un diamètre moyen compris entre 0.01 et 6 µm, de préférence entre 0.1 et 0.35 µm.

3. Composition selon l'une des revendications 1 ou 2, **caractérisée par le fait que** le(s) lipide(s) amphiphile(s) non-ionique(s) est (sont) choisi(s) dans le groupe formé par les éthers et les esters de polyglycérol linéaires ou ramifiés de formules (II) et (III) respectives:

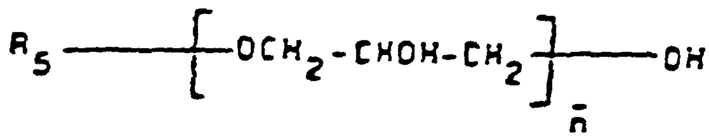

(II)

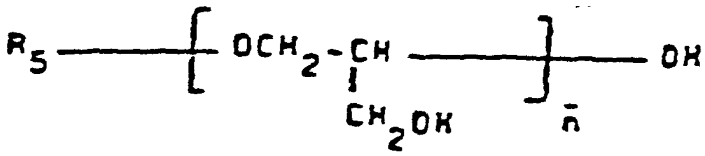 (III)

formules où $\bar{n}$ est une valeur statistique moyenne comprise entre 2 et 6 et où $R_5$ est:

1) ou bien une chaine aliphatique $R_6$ ou un reste $R'_6$ CO. $R_6$ étant un radical aliphatique, linéaire ou ramifié, en $C_{12}$ - $C_{18}$ et $R'_6$ étant un radical aliphatique, linéaire ou ramifié, en $C_{11}$-$C_{17}$;
2) ou bien un racical

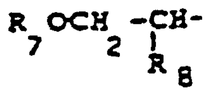

où $R_7$ et $R_8$ sont des radicaux $R_4$ ou $R'_6$ CO, identiques ou différents, $R_6$ et $R'_6$ ayant les significations définies ci-dessus.

**4.** Composition selon l'une des revendications 1 à 3, **caractérisée par le fait que** le(s) lipidique(s) amphiphile(s) non-ionique(s), qui constitue(nt) la phase lamellaire lipidique hydratée est (ou sont) associé(s) à au moins un stabilisant choisi dans le groupe formé par les stérols, les sels meno ou d'sodiques de glutamates d'acyle, le radical acyle étant en $C_{14}$ - $C_{22}$, et les esters phosphoriques d'alcools gras en $C_{12}$- $C_{18}$.

**5.** Composition selon l'une des revendications 1 à 4, **caractérisée par le fait que** le(s) lipide(s) amphiphile(s) non-ionique(s), qui constitue(nt) la phase lamellaire lipidique hydratée, est (sont) associé(s) à au moins un stabilisant anionique en une quantité au plus égale à 12 % en poids et/ou à au moins un stérol en une quantité au plus égale à 100 % en poids, la proportion étant calculée, dans les deux cas, par rapport au poids dudit (ou desdits) lipide(s) amphiphile(s) non-ionique(s).

**6.** Composition selon les revendications 3 et 4, prises en combinaison, dans laquelle le(s) lipide(s) amphiphile(s) non-ionique(s), qui constitue(nt) la phase lamellaire lipidique hydratée, comprend (ou comprennent) un éther ou un ester de polyglycérol de formule (II) ou (III), dans lequel $R_5$ est une chaîne aliphatique $R_6$ ou $R'_6$ CO, $R_5$ et $R'_5$ ayant les significations indiquées à la revendication 4, **caractérisée par le fait que** le(s)dit(s) lipide(s) non-ionique (s) est (sont) associé(s) à la fois à un stérol et à un stabilisant anionique.

**7.** Composition selon l'une des revendications 1 à 6, **caractérisée par le fait que** la phase lamellaire lipidique hydratée contient de l'eau en présence ou non d'un agent cosmétiquement ou pharmaceutiquement actif.

**8.** Composition selon la revendication 7, dans laquelle la phase lamellaire lipidique hydratée contient un agent actif, **caractérisée par le fait que** l'agent actif est choisi dans le groupe formé par les agents anti-chute ou de repousse des cheveux, les rétinoïdes et apparentés, les anti-inflammatoires, les anti-fongiques, les anti-séborrhéiques et les filtres solaires.

**9.** Composition selon l'une des revendications 1 à 8, **caractérisée par le fait que** l'agent tensio-actif cationique contenu dans la phase aqueuse continue est choisi dans le groupe formé par :

a) les halogénures de tétraalkylammonium ;
b) le chlorure de stéaramidopropyl-diméthyl (myristyl acétate) ammonium de formule :

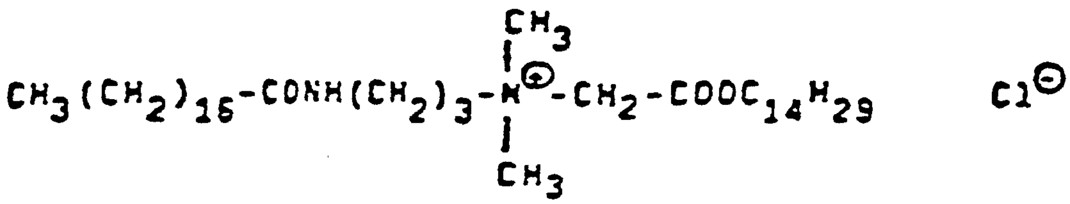

c) un sel d'ammonium quaternaire de formule :

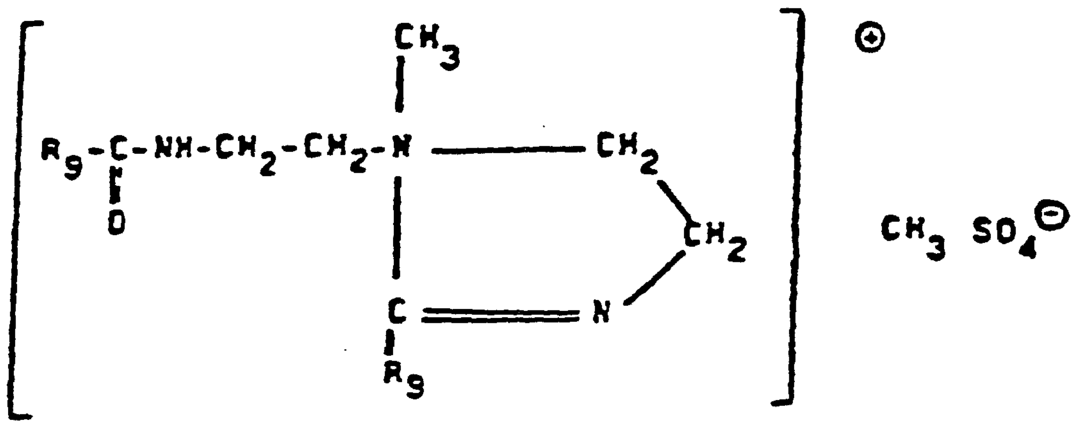

dans lequel $R_9$ désigne un mélange de radicaux alkényle et/ou alkyle en $C_{13}$ - $C_{21}$ dérivé des acides gras du suif.

**10.** Composition selon l'une des revendications 1 à 9, **caractérisée par le fait que** la protéine quaternisée est choisie dans le groupe formé par :

a) des hydrolysats de protéine animale portant sur la chaîne polypeptidique des groupements ammonium quaternaires comportant au moins un radical alkyle en $C_1$ - $C_{18}$;
b) des hydrolysats de protéine animale portant des groupements triméthylbenzylammonium ;
c) des hydrolysats de collagène porteurs de groupements triéthylammonium ;
d) des hydrolysats de collagène porteurs de groupements triméthylammonium et triméthylstéarylammonium ;
e) une protéine quaternisée résultant de la condensation de la cocamidopropyl-diméthyl-amine sur une protéine animale hydrolysée.

**11.** Composition selon l'une des revendications 1 à 10 **caractérisée par le fait qu'**elle contient à la fois au moins un agent tensio-actif cationique et au moins une protéine quaternisée.

**12.** Composition selon la revendication 11, **caractérisée par le fait qu'**elle contient d'une part, du chlorure de béhényltriméthylammonium et d'autre part, un hydrolysat de protéine animale porteur sur la chaîne polypeptidique de groupements ammonium quaternaires comportant au moins une chaîne alkyle en $C_1$ - $C_{18}$, la chaîne polypeptidique ayant un poids moléculaire moyen d'environ 12.000.

**13.** Composition selon l'une des revendications 1 à 12, **caractérisée par le fait que** la phase aqueuse continue contient au moins un additif pris dans le groupe formé par les conservateurs, les stabilisants, les colorants, les humectants, les adoucissants, les parfums et les épaississants.

**14.** Composition selon l'une des revendications 1 à 13, contenant au moins un agent tensio-actif cationique, **caractérisée par le fait que** ledit agent tensio-actif cationique représente de 0,1 à 6 %, du poids total de la composition.

**15.** Composition selon l'une des revendications 1 à 14,contenant au moins une protéine quaternisée, **caractérisée**

**par le fait que** ladite protéine quaternisée représente de 0,05 à 0,5 % du poids total de la composition.

**16.** Composition selon l'une des revendications 1 à 15, **caractérisée par le fait que** le (les) lipide (s) amphiphile (s) non-ionique (s) représente (nt) de 1 à 10 % en poids, de préférence de 3 à 10 % en poids par rapport au poids total de la composition.

**17.** Procédé de traitement des cheveux et du cuir chevelu, **caractérisé par le fait qu'**on applique sur la tête 20 à 40 g d'une composition selon l'une des revendications 1 à 16 qu'on laisse en contact pendant 1 à 15 mn, puis qu'on rince à l'eau.

**18.** Procédé de traitement des cheveux et du cuir chevelu, **caractérisé par le fait qu'**on applique sur la tête 5 à 10 g d'une composition selon l'une des revendications 1 à 16.

**Revendications pour les Etats contractants suivants : ES, GR**

**1.** Composition cosmétique pour le traitement des cheveux renfermant des lipides amphiphiles non-ioniques susceptibles de former une phase lamellaire lipidique hydratée insolubles dans l'eau, éventuellement associés à un stabilisant, sous forme de vésicules dispersées dans une phase aqueuse continue, **caractérisée par le fait que** ladite phase aqueuse contient :

1) au moins un agent tensio-actif cationique de formule :

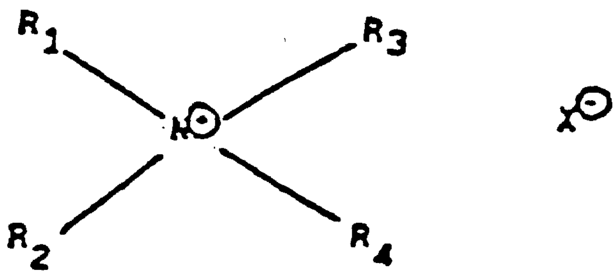

(I)

formule dans laquelle X est le chlore ou $CH_3SO\text{-}_4$ et $R_1$ est un radical alkyle en $C_1$ - $C_4$ et dans laquelle :

a) lorsque X est le chlore :

- ou bien $R_2$ et $R_3$ sont des radicaux alkyle en $C_1$ - $C_4$, identiques ou différents de $R_1$ et entre eux, et $R_4$ est un radical alkyle en $C_{16}$ - $C_{22}$ ;
- ou bien $R_2$ = $R_1$ et. dans ce cas :

soit $R_3$ = $R_4$ = radical alkyle en $C_{18}$ ;
soit $R_3$ = radical (alkyl $C_{17}$)amidopropyle et $R_4$ = radical (alkyl $C_{14}$)acétate et

b) lorsque X est $CH_3SO\text{-}_4$:

- $R_2$ désigne un radical (alkyl et/ou alkényl amidoéthyle, dans lequel le radical alkyle et/ou alkényle est un radical en $C_{13}$ - $C_{21}$ et dérive des acides gras du suif ;
- $R_3$ et $R_4$ forment ensemble avec l'azote un cycle 4,5-dihydroimidazole substitué en position 2 ; et/ou

2) au moins une protéine quaternisée constituée par un polypeptide modifié chimiquement portant, en bout de chaîne ou greffé sur celle-ci, au moins un groupement ammonium quaternaire qui contient au moins un groupe alkyle en $C_1$ - $C_{18}$, le polypeptide étant choisi parmi les hydrolysats de protéine animale, le (s) agent (s) tensio-actif(s) représentant de 0,05 à 10 % en poids par rapport au poids total de la composition, la (les) protéine (s) quaternisée (s) représentant de 0,05 à 3 % en poids par rapport au poids total de la composition, le (les) lipide (s) amphiphile (s) non ionique (s) représentant de 0,1 à 20 % en poids par rapport au poids total

de la composition.

**2.** Composition selon la revendication 1, **caractérisée par le fait que** les vésicules ont un diamètre moyen compris entre 0,01 et 5 μm, de préférence entre 0,1 et 0.35 μm.

**3.** Composition selon l'une des revendications 1 ou 2, **caractérisée par le fait que** le(s) lipide(s) amphiphile(s) non-ionique(s) est (sont) choisi(s) dans le groupe formé par les éthers et les esters de polyglycérol linéaires ou ramifiés de formules (II) et (III) respectives :

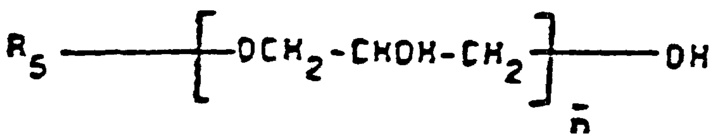

(II)

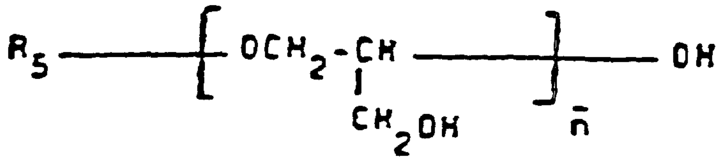

(III)

formules où $\bar{n}$ est une valeur statistique moyenne comprise entre 2 et 6 et où $R_5$ est :

1) ou bien une chaîne aliphatique $R_6$ ou un reste $R'_6$ CO, $R_6$ étant un radical aliphatique, linéaire ou ramifié, en $C_{12}$- $C_{18}$ et $R'_6$ étant un radical aliphatique, linéaire ou ramifié, en $C_{11}$-$C_{17}$ ;
2) ou bien un radical

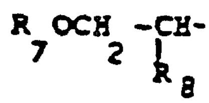

où $R_7$ et $R_8$ sont des radicaux $R_6$ ou $R'_6$ CO, identiques ou différents, $R_6$ et $R'_6$ ayant les significations définies ci-dessus.

**4.** Composition selon l'une des revendications 1 à 3, **caractérisée par le fait que** le(s) lipidique(s) amphiphile(s) non-ionique(s), qui constitue(nt) la phase lamellaire lipidique hydratée est (ou sont) associé(s) à au moins un stabilisant choisis dans le groupe formé par les stérols, les sels mono ou disodiques de glotamates d'acyle, le radical acyle étant en $C_{14}$ - $C_{22}$, et les esters phosphoriques d'alcools gras en $C_{12}$ - $C_{18}$.

**5.** Composition selon l'une des revendications 1 à 4, **caractérisée par le fait que** le(s) lipide(s) amphiphile(s) non-ionique(s), qui constitue(nt) la phase lamellaire lipidique hydratée, est (sont) associé(s) à au moins un stabilisant anionique en une quantité au plus égale à 12 % en poids et/ou à au moins un stérol en une quantité au plus égale à 100 % en poids, la proportion étant calculée, dans les deux cas, par rapport au poids dudit (ou desdits) lipide(s) amphiphile(s) non-ioniques(s).

**6.** Composition selon les revendications 3 et 4, prises en combinaison, dans laquelle le(s) lipide(s) amphiphile(s) non-ionique(s), qui constitue(nt) la phase lamellaire lipidique hydratée, comprend (ou comprennent) un éther ou un ester de polyglycérol de formule (II) ou (III), dans lequel $R_5$ est une chaîne aliphatique $R_5$ ou $R'_5$ CO, $R_6$ et $R'_6$ ayant les significations indiquées à la revendication 4, **caractérisée par le fait que** le(s)dit(s) lipids(s) non-ionique (s) est (sont) associé(s) à la fois à un stérol et à un stabilisant anionique.

**7.** Composition selon l'une des revendications 1 à 6, **caractérisée par le fait que** la phase lamellaire lipidique hydratée contient de l'eau en présence ou non d'un agent cosmétiquement ou pharmaceutiquement actif.

**8.** Composition selon la revendication 7, dans laquelle la phase lamellaire lipidique hydratée contient un agent actif, **caractérisée par le fait que** l'agent actif est choisi dans !e groupe formé par les agents anti-chute ou de repousse des cheveux, les rétinoïdes et apparentés, les anti-inflammatoires, les antifongiques, les anti-séborrhéiques et les filtres solaires.

**9.** Composition selon l'une des revendications 1 à 8, **caractérisée par le fait que** l'agent tensio-actif cationique contenu dans la phase aqueuse continue est choisi dans le groupe formé par :

a) les halogénures de tétraalkylammonium ;
b) le chlorure de stéaramidopropyl-diméthyl (myristyl acétate) ammonium de formule :

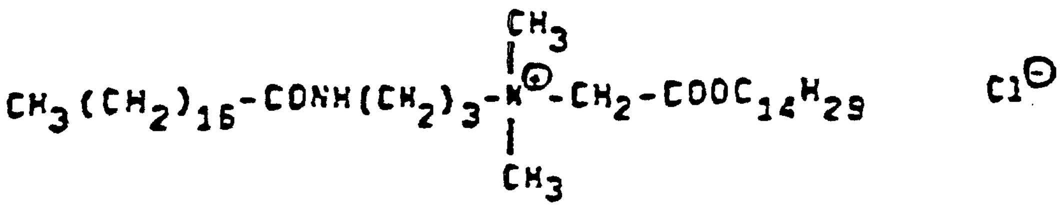

c) un sel d'ammonium quaternaire de formule :

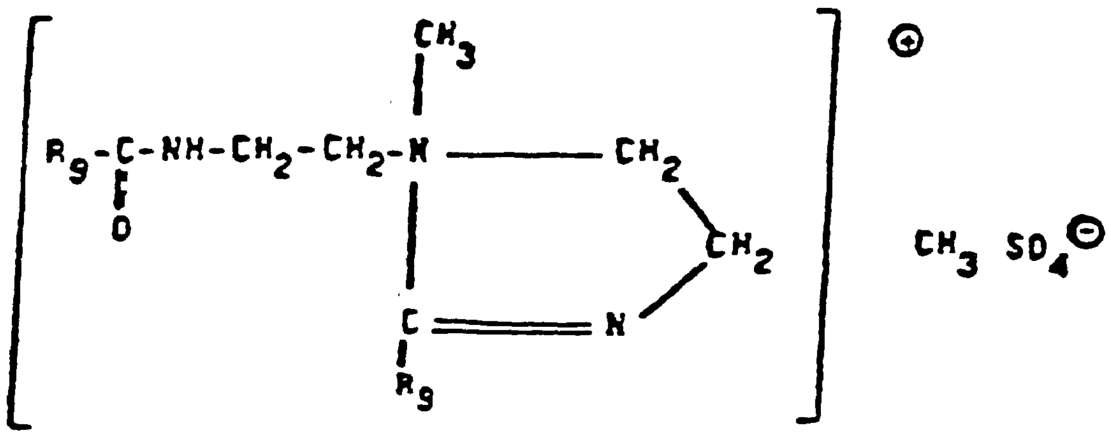

dans lequel $R_9$ désigne un mélange de radicaux alkényle et/ou alkyle en $C_{13}$ - $C_{21}$ dérivé des acides gras du suif.

**10.** Composition selon l'une des revendications 1 à 9, **caractérisée par le fait que** la protéine quaternisée est choisie dans le groupe formé par :

a) des hydrolysats de protéine animale portant sur la chaîne polypeptidique des groupements ammonium quaternaires comportant au moins un radical alkyle en $C_1$ - $C_{18}$;
b) des hydrolysats de protéine animale portant des groupements triméthylbenzylammonium ;
c) des hydrolysats de collagène porteurs de groupements triéthylammonium ;
d) des hydrolysats de collagène porteurs de groupements triméthylammonium et triméthylstéarylammonium ;
e) une protéine quaternisée résultant de la condensation de la cocamidopropyl-diméthyl-amine sur une protéine animale hydrolysée.

**11.** Composition selon l'une des revendications 1 à 10, **caractérisée par le fait qu'**elle contient à la fois au moins un agent tensio-actif cationique et au moins une protéine quaternisée.

**12.** Composition selon la revendication 11, **caractérisée par le fait qu'**elle contient d'une part, du chlorure de béhényltriméthylammonium et d'autre part, un hydrolysat de protéine animale porteur sur la chaîne polypeptidique de

groupements ammonium quaternaires comportant au moins une chaîne alkyle en $C_1$ - $C_{18}$, la chaîne polypeptidique ayant un poids moléculaire moyen d'environ 12.000.

**13.** Composition selon l'une des revendications 1 à 12, **caractérisée par le fait que** la phase aqueuse continue contient au moins un additif pris dans le groupe formé par les conservateurs, les stabilisants, les colorants, les humectants, les adoucissants, les parfums et les épaississants.

**14.** Composition selon l'une des revendications 1 à 13,contenant au moins un agent tensio-actif cationique, **caractérisée par le fait que** ledit agent tensio-actif cationique représente de 0,1 à 6 %, du poids total de la composition.

**15.** Composition selon l'une des revendications1 à 14,contenant au moins une protéine quaternisée, **caractérisée par le fait que** ladite protéine quaternisée représente de 0,05 à 0,5 % du poids total de la composition.

**16.** Composition selon l'une des revendications 1 à 15, **caractérisée par le fait que** le (les) lipide (s) amphiphile (s) non-ionique (s) représente (nt) de 1 à 10 % en poids, de préférence de 3 à 10 % en poids par rapport au poids total de la composition.

**17.** Procédé de traitement des cheveux et du cuir chevelu, **caractérisé par le fait qu'**on applique sur la tête 20 à 40 g d'une composition selon l'une des revendications 1 à 16 qu'on laisse en contact pendant 1 à 15 mn, puis qu'on rince à l'eau.

**18.** Procédé de traitement des cheveux et du cuir chevelu, **caractérisée par le fait qu'**on applique sur la tête 5 à 10 g d'une composition selon l'une des revendications 1 à 16.

**Patentansprüche**

**Patentansprüche für folgende Vertragsstaaten : AT, BE, CH, DE, FR, GB, IT, LI, NL, SE**

**1.** Kosmetisches oder pharmazeutisches Mittel zur Behandlung der Haare, welches, gegebenenfalls in Kombination mit einem Stabilisator, amphiphile, nichtionische Lipide, die eine lamellare, hydratisierte Lipidphase zu bilden vermögen und in Wasser unlöslich sind, in Form von Vesikeln, die in einer wässrigen, kontinuierlichen Phase dispergiert sind, umfasst,

**dadurch gekennzeichnet, daß** die wäßrige Phase enthält:

(1) mindestens ein kationisches oberflächenaktives Mittel der allgemeinen Formel

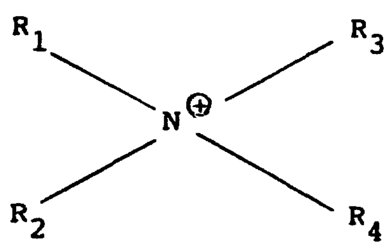

$X^{\ominus}$ (I)

worin X für Chlor oder $CH_3SO_4^-$ steht, und $R_1$ einen $C_1$ - $C_4$-Alkylrest bedeutet, und worin

a) wenn X für Chlor steht:
entweder $R_2$ und $R_3$ für $C_1$ - $C_4$-Alkylreste stehen, die zu $R_1$ und untereinander gleich oder verschieden sind und $R_4$ einen $C_{16}$ - $C_{22}$-Alkylrest bedeutet; oder

$R_2 = R_1$ ist, und
$R_3 = R_4 = C_{18}$- Alkyl ist; oder

$R_3$ = ($C_{17}$-Alkyl)-amidopropyl und
$R_4$ = ($C_{14}$-Alkyl)-acetat und

b) wenn X für $CH_3SO_4^-$ steht:

$R_2$ einen (Alkyl- und/oder Alkenyl)-amidoethylrest bedeutet, worin der Alkyl- und/oder Alkenylrest ein $C_{13}$ - $C_{21}$ -Rest, abgeleitet von Talg-Fettsäuren, ist;
$R_3$ und $R_4$ zusammen mit dem Stickstoffatom einen 4,5-Dihydroimidazolring, der in Position 2 substituiert ist, bilden;
und/oder

(2) mindestens ein quaternisiertes Protein, welches aus einem chemisch modifizierten Polypeptid gebildet wird, das am Kettenende oder aufgepropft mindestens eine quaternäre Ammoniumgruppe trägt, die mindestens eine $C_1$ - $C_{18}$-Alkylkette enthält, wobei das Polypeptid ausgewählt ist unter Hydrolysaten tierischer Proteine,

wobei das (die) oberflächenaktive (n) Mittel 0.05 bis 10 Gew.-%, bezogen auf das Gesamtgewicht des Mittels, ausmacht (ausmachen), das (die) quaternisierte(n) Protein(e) 0,05 bis 3 Gew.-%, bezogen auf das Gesamtgewicht des Mittels, ausmacht(ausmachen), und das (die) amphiphile(n) ionische(n) Lipid(e) 0,1 bis 20 Gew.-%, bezogen auf das Gesamtgewicht des Mittels, ausmacht (ausmachen).

**2.** Mittel nach Anspruch 1, **dadurch gekennzeichnet, daß** die Vesikel einen mittleren Durchmesser von 0,01 bis 5 µ, vorzugsweise von 0,1 bis 0,35 µ, aufweisen.

**3.** Mittel nach einem der Ansprüche loder 2, **dadurch gekennzeichnet, daß** das (die) nicht-ionische(n) amphiphile (n) Lipid(e) ausgewählt ist (sind) unter geradkettigen oder verzweigten Polyglycoläthern und -estern, der allgemeinen Formeln (II) bzw. (III):

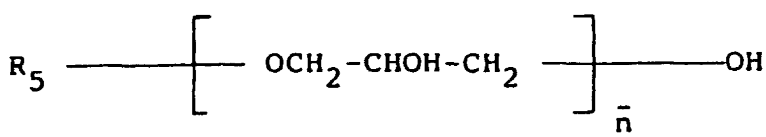

(II)

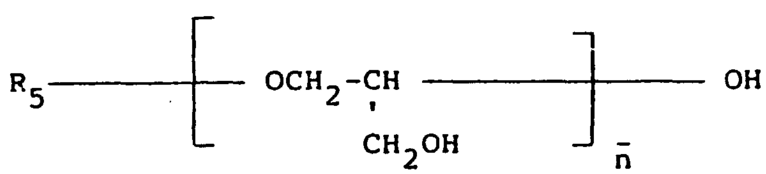

(III)

worin $\bar{n}$ einen statistischen Mittelwert zwischen 2 und 6 bedeutet und $R_5$

1) entweder für eine aliphatische Kette $R_6$ oder für einen Rest $R'_6CO$ steht, worin $R_6$ einen geradkettigen oder verzweigten aliphatischen $C_{12}$ - $C_{18}$-Rest bedeutet und $R'_6$ einen geradkettigen oder verzweigten aliphatischen $C_{11}$ - $C_{17}$-Rest bedeutet;
2) oder einen Rest

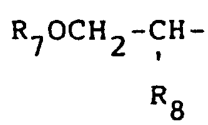

bedeutet, worin $R_7$ und $R_8$ gleich oder verschieden sind und den Rest $R_6$ oder $R'_6$ CO bedeuten, wobei $R_6$ und $R'_6$ die oben angegebenen Bedeutungen besitzen.

**4.** Mittel nach einem der Ansprüche 1 bis 3, **dadurch gekennzeichnet, daß** das (die) nichtionische(n) amphiphile (n) Lipid(e), das (die) die lamellare, hydratisierte Lipidphase bildet (bilden),mit mindestens einem Stabilisator kombiniert ist (sind), der ausgewählt ist unter Sterolen, Mono- oder D inatriumsalzen von Acylglutamaten, wobei der Acylrest ein $C_{14}$ - $C_{22}$-Rest ist, und Phosphorsäureestern von $C_{12}$ - $C_{16}$-Fettalkoholen.

**5.** Mittel nach einem der Ansprüche 1-4, **dadurch gekennzeichnet, daß** das (die) nichtionische(n) amphiphile(n) Lipid(e), das (die) die lamellare, hydratisierte Lipidphase bildet (bilden), kombiniert ist (sind) mit mindestens einem anionischen Stabilisator in einer Menge von höchstens 12 Gew.-% und/oder mindestens einem Sterol in einer Menge von höchstens 100 Gew.-%, wobei der Anteil jeweils in Bezug auf das Gewicht des (oder der) amphiphilen nichtionischen Lipids (Lipide) angegeben ist.

**6.** Mittel nach einer Kombination der Ansprüche 3 und 4, worin das (die) amphiphile(n) nichtionische(n) Lipid(e), das (die) die lamellare, hydratisierte Lipidphase bildet (bilden), einen Polyglycoläther- oder -ester der allgemeinen Formel (II) oder (III) umfaßt (umfassen), worin $R_5$ einen aliphatischen Rest $R_6$ oder $R'_6$CO bedeutet, wobei $R_6$ und $R'_6$ die in Anspruch 4 angegebenen Bedeutungen besitzen, **dadurch gekennzeichnet, daß** das (die) nichtionische (n) Lipid(e) mit einem Sterol und zugleich mit einem anionischen Stabilisator kombiniert ist (sind).

**7.** Mittel nach einem der Ansprüche 1-6, **dadurch gekennzeichnet, daß** die lamellare, hydratisierte Lipidphase Wasser und ggf. einen kosmetisch oder pharmazeutisch aktiven Wirkstoff enthält.

**8.** Mittel nach Anspruch 7, wobei die lamellare, hydratisierte Lipidphase einen aktiven Wirkstoff enthält, **dadurch gekennzeichnet, daß** der aktive Wirkstoff ausgewählt ist unter Mitteln gegen Haarausfall oder Mitteln zur Förderung des Nachwachsens der Haare, Retinoiden und verwandten Verbindungen, anti-inflammatorischen Mitteln, Antifungusmitteln, Antiseborrhoemitteln und Sonnenfiltern.

**9.** Mittel nach einem der Ansprüche 1-8, **dadurch gekennzeichnet, daß** das in der wäßrigen Phase enthaltene kationische oberflächenaktive Mittel ausgewählt ist unter

a) Tetraalkylammoniumhalogeniden;

b) Stearamidopropyl-dimethyl(myristylacetat)-ammoniumchlorid der allgemeinen Formel

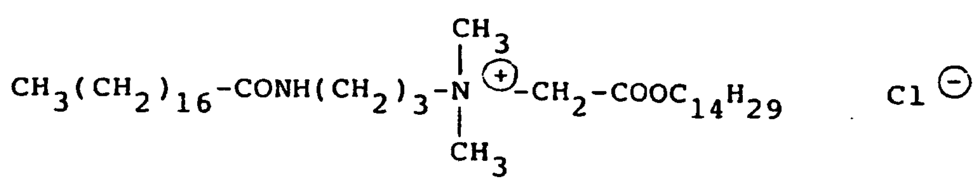

c) einem quaternären Ammoniumsalz der allgemeinen Formel

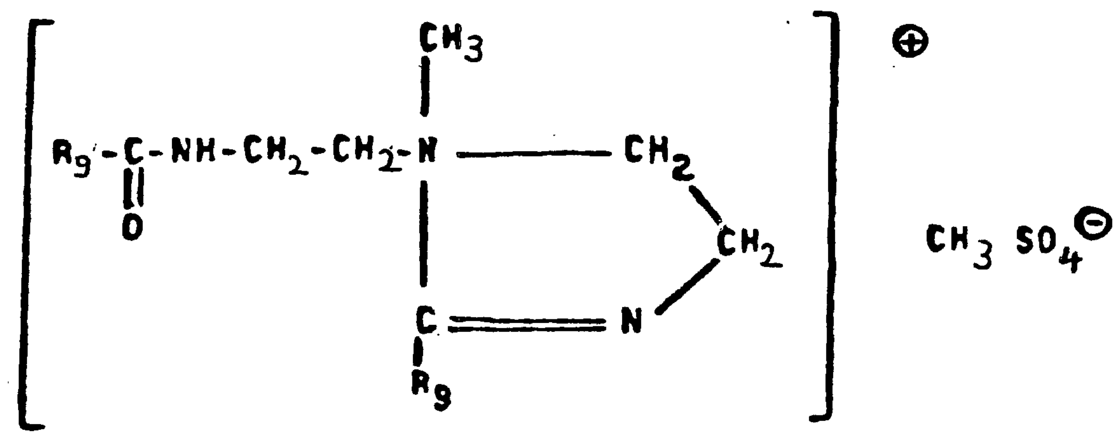

worin $R_9$ ein Gemisch aus $C_{13}$ - $C_{21}$-Alkenyl- und/oder Alkylresten darstellt, welche aus Talg-Fettsäuren abgeleitet sind.

**10.** Mittel nach einem der Ansprüche 1-9 , **dadurch gekennzeichnet, daß** das quaternisierte Protein ausgewählt ist unter

a) tierischen Proteinhydrolysaten, welche auf der Polypeptidkette quaternäre Ammoniumgruppen tragen, die mindestens einen $C_1$ - $C_{18}$-Alkylrest umfassen;

b) tierischen Proteinhydrolysaten, welche Trimethylbenzylammoniumgruppen tragen;

c) Collagenhydrolysaten mit Triethylammoniumgruppen;

d) Collagenhydrolysaten mit Trimethylammonium- und Trimethylstearylammoniumgruppen;

e) einem quaternisierten Protein, gebildet durch Kondensation von Cocamidopropyl-dimethylamin mit einem hydrolisierten tierischen Protein.

**11.** Mittel nach einem der Ansprüche 1-10, **dadurch gekennzeichnet, daß** es zugleich mindestens ein kationisches, oberflächenaktives Mittel und mindestens ein quaternisiertes Protein enthält.

**12.** Mittel nach Anspruch 11, **dadurch gekennzeichnet, daß** es Behenyltrimethylammoniumchlorid und ein tierisches Proteinhydrolysat umfaßt, welches auf der Polypeptidkette quaternäre Ammoniumgruppen trägt, die mindestens eine $C_1$ - $C_{18}$-Alkylkette umfassen, wobei die Polypeptidkette ein mittleres Molekulargewicht von etwa 12.000 besitzt.

**13.** Mittel nach einem der Ansprüche 1- 12 **dadurch gekennzeichnet, daß** die kontinuierliche wäßrige Phase mindestens ein Additiv enthält, das ausgewählt ist unter Konservierungsmitteln, Stabilisatoren, Farbstoffen, Feuchthaltemitteln, weichmachenden Mitteln, Parfüms und Verdickungsmitteln.

**14.** Mittel nach einem der Ansprüche 1-13, das mindestens ein kationisches, oberflächenaktives Mittel enthält, **dadurch gekennzeichnet, daß** das kationische, oberflächenaktive Mittel in einem Anteil von 0,1-6 Gew.-%, bezogen auf das Gesamtgewicht des Mittels, enthalten ist.

**15.** Mittel nach einem der Ansprüche 1-14, welches mindestens ein quaternisiertes Protein enthält, **dadurch gekennzeichnet, daß** das quaternisierte Protein in einem Anteil von 0,05-0,5 Gew.-%, bezogen auf das Gesamtgewicht des Mittels, enthalten ist.

**16.** Mittel nach einem der Ansprüche 1 bis 15,
**dadurch gekennzeichnet, daß** das (die) amphiphile(n), nicht-ionische(n) Lipid(e) 1 bis 10 Gew.-%, vorzugsweise

3 bis 10 Gew.-%, bezogen auf das Gesamtgewicht des Mittels, ausmacht (ausmachen).

**17.** Verfahren zur Behandlung der Haare und der Kopfhaut, **dadurch gekennzeichnet, daß** man auf den Kopf 20-40 Gramm eines Mittels nach einem der Ansprüche 1- 16 aufträgt, dieses 1 bis 15 Minuten in Kontakt hält und anschließend mit Wasser spült.

**18.** Verfahren zur Behandlung der Haare und der Kopfhaut, **dadurch gekennzeichnet, daß** man auf den Kopf 5 bis 10 Gramm eines Mittels nach einem der Ansprüche 1-16 aufträgt.

**Patentansprüche für folgende Vertragsstaaten : ES, GR**

**1.** Kosmetisches Mittel zur Behandlung der Haare, welches, gegebenenfalls in Kombination mit einem Stabilisator, amphiphile, nichtionische Lipide, die eine lamellare, hydratisierte Lipidphase zu bilden vermögen und in Wasser unlöslich sind, in Form von Vesikeln, die in einer wässrigen, kontinuierlichen Phase dispergiert sind, umfasst, **dadurch gekennzeichnet , daß** die wäßrige Phase enthält:

(1) mindestens ein kationisches oberflächenaktives Mittel der allgemeinen Formel

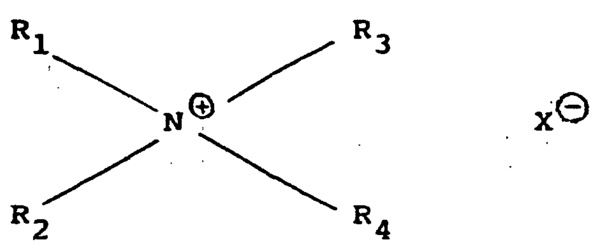

(I)

worin X für Chlor oder $CH_3SO_4^-$ steht, und $R_1$ einen $C_1$ - $C_4$-Alkylrest bedeutet, und worin

a) wenn X für Chlor steht:
entweder $R_2$ und $R_3$ für $C_1$ - $C_4$-Alkylreste stehen, die zu $R_1$ und untereinander gleich oder verschieden sind und $R_4$ einen $C_{16}$ - $C_{22}$-Alkylrest bedeutet; oder

$R_2 = R_1$ ist, und
$R_3 = R_4 = C_{18}$- Alkyl ist; oder
$R_3$ = ($C_{17}$-Alkyl)-amidopropyl und
$R_4$ = ($C_{14}$-Alkyl)-acetat und

b) wenn X für $CH_3SO_4^-$ steht:

$R_2$ einen (Alkyl- und/oder Alkenyl)-amidoethylrest bedeutet, worin der Alkyl- und/oder Alkenylrest ein $C_{13}$ - $C_{21}$-Rest, abgeleitet von Talg-Fettsäuren, ist;
$R_3$ und $R_4$ zusammen mit dem Stickstoffatom einen 4,5-Dihydroimidazolring, der in Position 2 substituiert ist, bilden;
und/oder

(2) mindestens ein quaternisiertes Protein, welches aus einem chemisch modifizierten Polypeptid gebildet wird, das am Kettenende oder aufgepropft mindestens eine quaternäre Ammoniumgruppe trägt, die mindestens eine $C_1$ - $C_{18}$-Alkylkette enthält, wobei das Polypeptid ausgewählt ist unter Hydrolysaten tierischer Proteine,

wobei das (die) oberflächenaktive (n) Mittel 0.05 bis 10 Gew.-%, bezogen auf das Gesamtgewicht des Mittels, ausmacht (ausmachen), das (die) quaternisierte(n) Protein(e) 0,05 bis 3 Gew.-%, bezogen auf das Gesamtgewicht des Mittels, ausmacht(ausmachen), und das (die) amphiphile(n) ionische(n) Lipid(e) 0,1 bis 20 Gew.-%, bezogen

auf das Gesamtgewicht des Mittels, ausmacht (ausmachen).

2. Mittel nach Anspruch 1, **dadurch gekennzeichnet, daß** die Vesikel einen mittleren Durchmesser von 0,01 bis 5 µ, vorzugsweise von 0,1 bis 0,35 µ, aufweisen.

3. Mittel nach einem der Ansprüche loder 2, **dadurch gekennzeichnet, daß** das (die) nicht-ionische(n) amphiphile (n) Lipid(e) ausgewählt ist (sind) unter geradkettigen oder verzweigten Polyglycoläthern und -estern, der allgemeinen Formeln (II) bzw. (III):

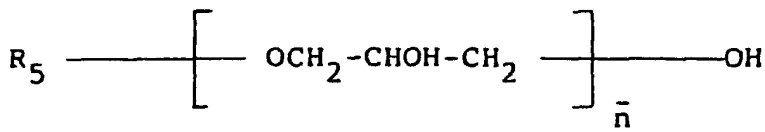

(II)

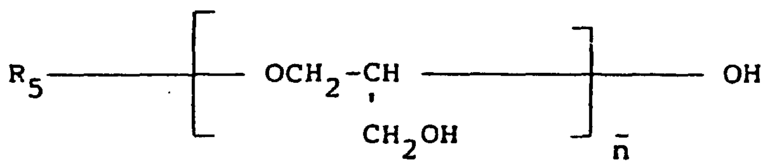

(III)

worin $\bar{n}$ einen statistischen Mittelwert zwischen 2 und 6 bedeutet und $R_5$

1) entweder für eine aliphatische Kette $R_6$ oder für einen Rest $R'_6CO$ steht, worin $R_6$ einen geradkettigen oder verzweigten aliphatischen $C_{12}$ - $C_{18}$-Rest bedeutet und $R'_6$ einen geradkettigen oder verzweigten aliphatischen $C_{11}$ - $C_{17}$-Rest bedeutet;
2) oder einen Rest

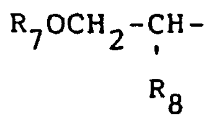

bedeutet, worin $R_7$ und $R_8$ gleich oder verschieden sind und den Rest $R_6$ oder $R'_6CO$ bedeuten, wobei $R_6$ und $R'_6$ die oben angegebenen Bedeutungen besitzen.

4. Mittel nach einem der Ansprüche 1 bis 3, **dadurch gekennzeichnet, daß** das (die) nichtionische(n) amphiphile (n) Lipid(e), das (die) die lamellare, hydratisierte Lipidphase bildet (bilden),mit mindestens einem Stabilisator kombiniert ist (sind), der ausgewählt ist unter Sterolen, Mono- oder D inatriumsalzen von Acylglutamaten, wobei der Acylrest ein $C_{14}$ - $C_{22}$-Rest ist, und Phosphorsäureestern von $C_{12}$ - $C_{16}$-Fettalkoholen.

5. Mittel nach einem der Ansprüche 1-4, **dadurch gekennzeichnet, daß** das (die) nichtionische(n) amphiphile(n) Lipid(e), das (die) die lamellare, hydratisierte Lipidphase bildet (bilden), kombiniert ist (sind) mit mindestens einem anionischen Stabilisator in einer Menge von höchstens 12 Gew.-% und/oder mindestens einem Sterol in einer Menge von höchstens 100 Gew.-%, wobei der Anteil jeweils in Bezug auf das Gewicht des (oder der) amphiphilen nichtionischen Lipids (Lipide) angegeben ist.

6. Mittel nach einer Kombination der Ansprüche 3 und 4, worin das (die) amphiphile(n) nichtionische(n) Lipid(e), das (die) die lamellare, hydratisierte Lipidphase bildet (bilden), einen Polyglycoläther- oder -ester der allgemeinen Formel (II) oder (III) umfaßt (umfassen), worin $R_5$ einen aliphatischen Rest $R_6$ oder $R'_6CO$ bedeutet, wobei $R_6$ und $R'_6$ die in Anspruch 4 angegebenen Bedeutungen besitzen, **dadurch gekennzeichnet, daß** das (die) nichtionische (n) Lipid(e) mit einem Sterol und zugleich mit einem anionischen Stabilisator kombiniert ist (sind).

**7.** Mittel nach einem der Ansprüche 1-6, **dadurch gekennzeichnet, daß** die lamellare, hydratisierte Lipidphase Wasser und ggf. einen kosmetisch oder pharmazeutisch aktiven Wirkstoff enthält.

**8.** Mittel nach Anspruch 7, wobei die lamellare, hydratisierte Lipidphase einen aktiven Wirkstoff enthält, **dadurch gekennzeichnet, daß** der aktive Wirkstoff ausgewählt ist unter Mitteln gegen Haarausfall oder Mitteln zur Förderung des Nachwachsens der Haare, Retinoiden und verwandten Verbindungen, anti-inflammatorischen Mitteln, Antifungusmitteln, Antiseborrhoemitteln und Sonnenfiltern.

**9.** Mittel nach einem der Ansprüche 1-8, **dadurch gekennzeichnet, daß** das in der wäßrigen Phase enthaltene kationische oberflächenaktive Mittel ausgewählt ist unter

a) Tetraalkylammoniumhalogeniden;

b) Stearamidopropyl-dimethyl(myristylacetat)-ammoniumchlorid der allgemeinen Formel

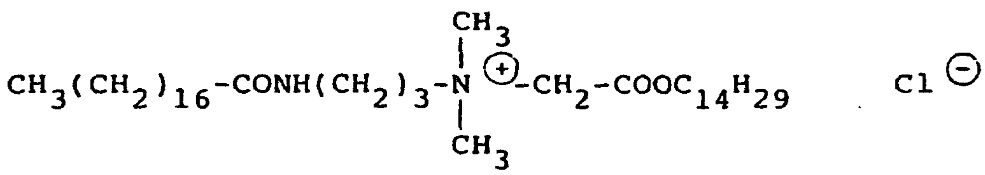

c) einem quaternären Ammoniumsalz der allgemeinen Formel

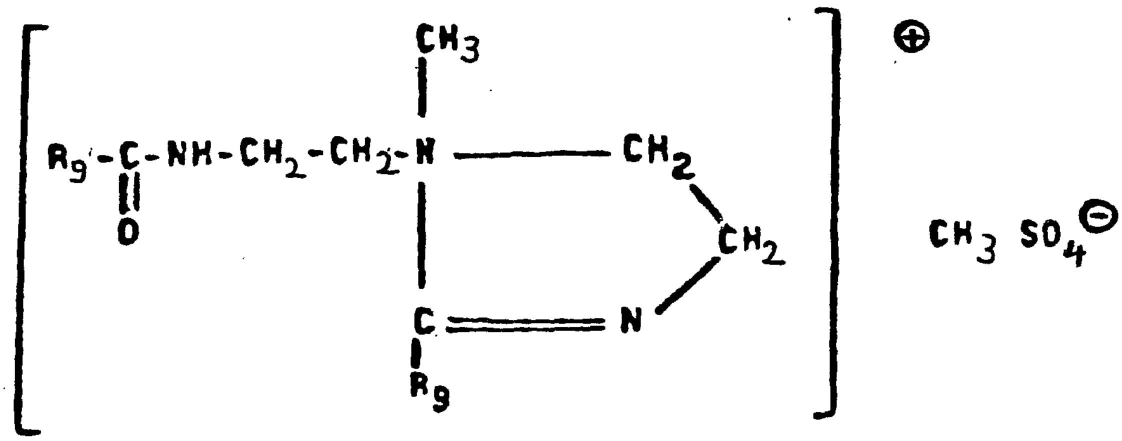

worin $R_9$ ein Gemisch aus $C_{13}$ - $C_{21}$-Alkenyl- und/oder Alkylresten darstellt, welche aus Talg-Fettsäuren abgeleitet sind.

**10.** Mittel nach einem der Ansprüche 1-9 , **dadurch gekennzeichnet, daß** das quaternisierte Protein ausgewählt ist unter

a) tierischen Proteinhydrolysaten, welche auf der Polypeptidkette quaternäre Ammoniumgruppen tragen, die mindestens einen $C_1$ - $C_{18}$-Alkylrest umfassen;

b) tierischen Proteinhydrolysaten, welche Trimethylbenzylammoniumgruppen tragen;

c) Collagenhydrolysaten mit Triethylammoniumgruppen;

d) Collagenhydrolysaten mit Trimethylammonium- und Trimethylstearylammoniumgruppen;

e) einem quaternisierten Protein, gebildet durch Kondensation von Cocamidopropyl-dimethylamin mit einem hydrolisierten tierischen Protein.

**11.** Mittel nach einem der Ansprüche 1-10, **dadurch gekennzeichnet, daß** es zugleich mindestens ein kationisches, oberflächenaktives Mittel und mindestens ein quaternisiertes Protein enthält.

**12.** Mittel nach Anspruch 11, **dadurch gekennzeichnet, daß** es Behenyltrimethylammoniumchlorid und ein tierisches Proteinhydrolysat umfaßt, welches auf der Polypeptidkette quaternäre Ammoniumgruppen trägt, die mindestens eine $C_1$ - $C_{18}$-Alkylkette umfassen, wobei die Polypeptidkette ein mittleres Molekulargewicht von etwa 12.000 besitzt.

**13.** Mittel nach einem der Ansprüche 1- 12 **dadurch gekennzeichnet, daß** die kontinuierliche wäßrige Phase mindestens ein Additiv enthält, das ausgewählt ist unter Konservierungsmitteln, Stabilisatoren, Farbstoffen, Feuchthaltemitteln, weichmachenden Mitteln, Parfüms und Verdickungsmitteln.

**14.** Mittel nach einem der Ansprüche 1-13, das mindestens ein kationisches, oberflächenaktives Mittel enthält, **dadurch gekennzeichnet, daß** das kationische, oberflächenaktive Mittel in einem Anteil von 0,1-6 Gew.-%, bezogen auf das Gesamtgewicht des Mittels, enthalten ist.

**15.** Mittel nach einem der Ansprüche 1-14, welches mindestens ein quaternisiertes Protein enthält, **dadurch gekennzeichnet, daß** das quaternisierte Protein in einem Anteil von 0,05-0,5 Gew.-%, bezogen auf das Gesamtgewicht des Mittels, enthalten ist.

**16.** Mittel nach einem der Ansprüche 1 bis 15,
**dadurch gekennzeichnet, daß** das (die) amphiphile(n), nicht-ionische(n) Lipid(e) 1 bis 10 Gew.-%, vorzugsweise 3 bis 10 Gew.-%, bezogen auf das Gesamtgewicht des Mittels, ausmacht (ausmachen).

**17.** Verfahren zur Behandlung der Haare und der Kopfhaut,
**dadurch gekennzeichnet, daß** man auf den Kopf 20-40 Gramm eines Mittels nach einem der Ansprüche 1- 16 aufträgt, dieses 1 bis 15 Minuten in Kontakt hält und anschließend mit Wasser spült.

**18.** Verfahren zur Behandlung der Haare und der Kopfhaut,
**dadurch gekennzeichnet, daß** man auf den Kopf 5 bis 10 Gramm eines Mittels nach einem der Ansprüche 1-16 aufträgt.

**Claims**

**Claims for the following Contracting States : AT, BE, CH, DE, FR, GB, IT, LI, NL, SE**

**1.** Cosmetic or pharmaceutical composition for the treatment of the hair comprising nonionic amphiphilic lipids capable of forming a hydrated lamellar lipid phase, insoluble in water, optionally combined with a stabilising agent, in the form of vesicles dispersed in a continuous aqueous phase, **characterised in that** the said aqueous phase contains:

1) at least one cationic surface-active agent having the formula:

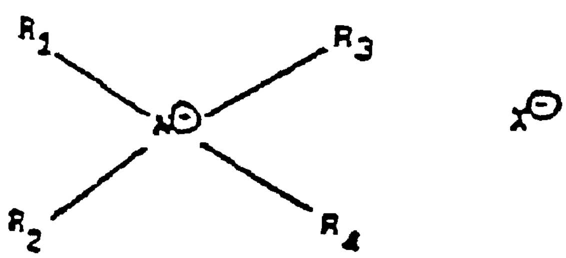

(I)

in which formula X is chlorine or $CH_3SO_4^-$ and $R_1$ is a $C_1$-$C_4$ alkyl radical and in which formula:

a) when X is chlorine:

- either $R_2$ and $R_3$ are $C_1$-$C_4$ alkyl radicals, identical or different from $R_1$ and each other, and $R_4$ is a $C_{16}$-$C_{22}$ alkyl radical;
- or $R_2$ = $R_1$, and in this case:

either $R_3$ = $R_4$ = $C_{18}$ alkyl radical;
or $R_3$ = ($C_{17}$ alkyl)amidopropyl radical and
$R_4$ = ($C_{14}$ alkyl) acetate radical, and

b) when X is $CH_3SO_4^-$:

- $R_2$ represents an (alkyl and/or alkenyl)amidoethyl radical in which the alkyl and/or alkenyl radical is a $C_{13}$-$C_{21}$ radical and is derived from tallow fatty acids;
- $R_3$ and $R_4$ together with the nitrogen form a 4,5-dihydroimidazole ring substituted in position 2; and/or

2) at least one quaternised protein consisting of a chemically modified polypeptide bearing, at the end of the chain or grafted onto the latter, at least one quaternary ammonium group which contains at least one $C_1$-$C_{18}$ alkyl group, the polypeptide being selected from animal protein hydrolysates, the surface-active agent(s) representing from 0.05 to 10 % by weight relative to the total weight of the composition, the quaternised protein (s) representing from 0.05 to 3 % by weight relative to the total weight of the composition and the nonionic amphiphilic lipid(s) representing from 0.1 to 20 % by weight relative to the total weight of the composition.

**2.** Composition according to Claim 1, **characterised in that** the vesicles have an average diameter of between 0.01 and 5 μm, and preferably between 0.1 and 0.35 μm.

**3.** Composition according to either of Claims 1 and 2, **characterised in that** the nonionic amphiphilic lipid(s) is/are selected from the group consisting of linear or branched ethers and esters of polyglycerol having the respective formulae (II) and (III):

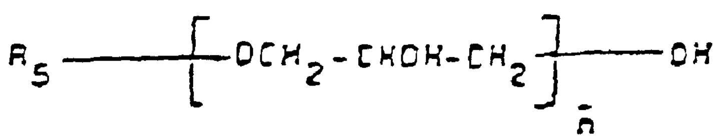

(II)

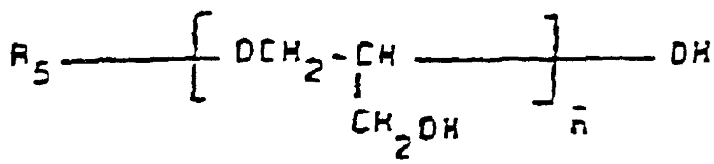

(III)

in which formulae $\bar{n}$ is an average statistical value of between 2 and 6 and $R_5$ is:

1) either an aliphatic chain $R_6$ or a residue $R'_6CO$, $R_6$ being a linear or branched $C_{12}$-$C_{18}$ aliphatic radical and $R'_6$ being a linear or branched $C_{11}$-$C_{17}$ aliphatic radical;
2) or a radical

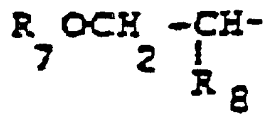

where $R_7$ and $R_8$ are radicals $R_6$ or $R'_6CO$, identical or different, $R_6$ and $R'_6$ having the meanings defined above.

**4.** Composition according to one of Claims 1 to 3, **characterised in that** the nonionic amphiphilic lipid(s) which constitute(s) the hydrated lamellar lipid phase is/are combined with at least one stabilising agent selected from the group consisting of sterols, mono- or disodium salts of acylglutamates, the acyl radical being a $C_{14}$-$C_{22}$ radical, and phosphoric esters of $C_{12}$-$C_{16}$ fatty alcohols.

**5.** Composition according to one of Claims 1 to 4, **characterised in that** the nonionic amphiphilic lipid(s) which constitute(s) the hydrated lamellar lipid phase is/are combined with at least one anionic stabilising agent in an amount at most equal to 12 % by weight and/or with at least one sterol in an amount at most equal to 100 % by weight, the proportion being calculated, in both cases, relative to the weight of the said nonionic amphiphilic lipid(s).

**6.** Composition according to Claims 3 and 4 taken in combination, in which the nonionic amphiphilic lipid(s) which constitute(s) the hydrated lamellar lipid phase comprise(s) an ether or ester of polyglycerol of formula (II) or (III) in which $R_5$ is an aliphatic chain $R_6$ or $R'_6CO$, $R_6$ and $R'_6$ having the meanings stated in Claim 3, **characterised in that** the said nonionic lipid(s) is/are combined with both a sterol and an anionic stabilising agent.

**7.** Composition according to one of Claims 1 to 6, **characterised in that** the hydrated lamellar lipid phase contains water in the presence or absence of a cosmetically or pharmaceutically active agent.

**8.** Composition according to Claim 7, in which the hydrated lamellar lipid phase contains an active agent, **characterised in that** the active agent is selected from the group consisting of agents combating hair loss or promoting hair regrowth, retinoids and related compounds, anti-inflammatory agents, antifungal agents, anti-seborrhoeic agents and sunscreen agents.

**9.** Composition according to one of Claims 1 to 8, **characterised in that** the cationic surface-active agent contained in the continuous aqueous phase is selected from the group consisting of:

a) tetraalkylammonium halides;
b) stearamidopropyldimethyl(myristyl acetate)-ammonium chloride having the formula:

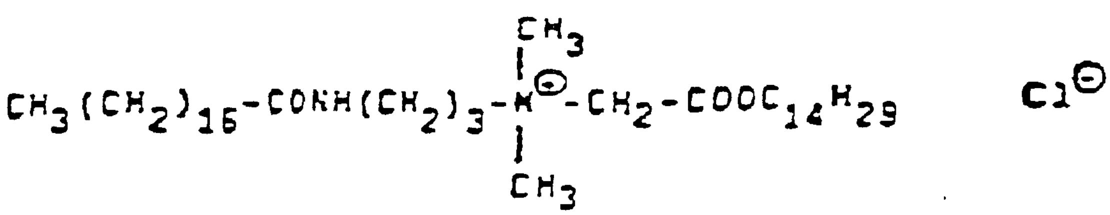

c) a quaternary ammonium salt having the formula:

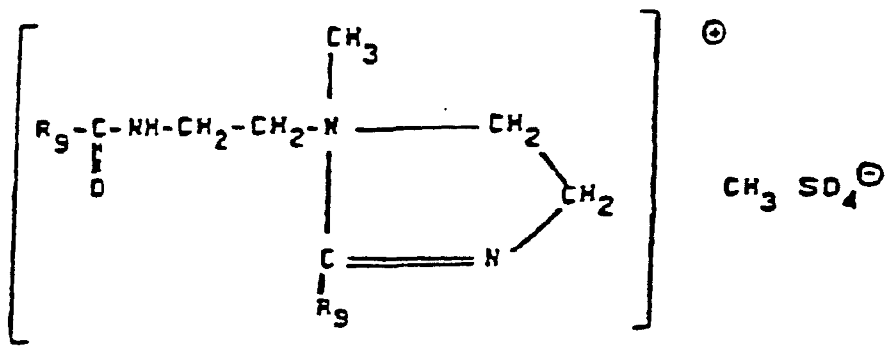

in which $R_9$ represents a mixture of $C_{13}$-$C_{21}$ alkenyl and/or alkyl radicals which is derived from tallow fatty acids.

**10.** Composition according to one of Claims 1 to 9, **characterised in that** the quaternised protein is selected from the group consisting of:

a) animal protein hydrolysates bearing on the polypeptide chain quaternary ammonium groups containing at least one $C_1$-$C_{18}$ alkyl radical;
b) animal protein hydrolysates bearing trimethylbenzylammonium groups;
c) collagen hydrolysates bearing triethylammonium groups;
d) collagen hydrolysates bearing trimethylammonium and trimethylstearylammonium groups;
e) a quaternised protein resulting from the condensation of cocamidopropyldimethylamine with a hydrolysed animal protein.

**11.** Composition according to one of Claims 1 to 10, **characterised in that** it contains both at least one cationic surface-active agent and at least one quaternised protein.

**12.** Composition according to Claim 11, **characterised in that** it contains, on the one hand behenyltrimethylammonium chloride, and on the other hand an animal protein hydrolysate bearing on the polypeptide chain quaternary ammonium groups containing at least one $C_1$-$C_{18}$ alkyl chain, the polypeptide chain having an average molecular weight of about 12,000.

**13.** Composition according to one of Claims 1 to 12, **characterised in that** the continuous aqueous phase contains at least one additive selected from the group consisting of preservatives, stabilising agents, colourings, humectants, emollients, fragrances and thickening agents.

**14.** Composition according to one of Claims 1 to 13, containing at least one cationic surface-active agent, **characterised in that** the said cationic surface-active agent represents from 0.1 to 6 % of the total weight of the composition.

**15.** Composition according to one of Claims 1 to 14, containing at least one quaternised protein, **characterised in that** the said quaternised protein represents from 0.05 to 0.5 % of the total weight of the composition.

**16.** Composition according to one of Claims 1 to 15, **characterised in that** the nonionic amphiphilic lipid(s) represent (s) from 1 to 10 % by weight, and preferably from 3 to 10 % by weight, relative to the total weight of the composition.

**17.** Process for the treatment of the hair and scalp, **characterised in that** 20 to 40 g of a composition according to one of Claims 1 to 16 are applied on the head, **in that** the composition is left in contact for 1 to 15 min and **in that** the head is then rinsed with water.

**18.** Process for the treatment of the hair and scalp, **characterised in that** 5 to 10 g of a composition according to one of Claims 1 to 16 are applied on the head.

**Claims for the following Contracting States : ES, GR**

**1.** Cosmetic composition for the treatment of the hair comprising nonionic amphiphilic lipids capable of forming a hydrated lamellar lipid phase, insoluble in water, optionally combined with a stabilising agent, in the form of vesicles dispersed in a continuous aqueous phase, **characterised in that** the said aqueous phase contains:

1) at least one cationic surface-active agent having the formula:

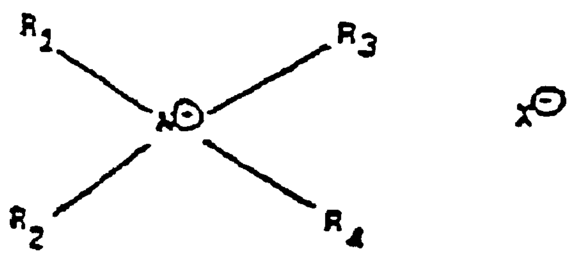

(I)

in which formula X is chlorine or $CH_3SO_4^-$ and $R_1$ is a $C_1$-$C_4$ alkyl radical and in which formula:

a) when X is chlorine:

- either $R_2$ and $R_3$ are $C_1$-$C_4$ alkyl radicals, identical or different from $R_1$ and each other, and $R_4$ is a $C_{16}$-$C_{22}$ alkyl radical;
- or $R_2 = R_1$, and in this case:

either $R_3 = R_4 = C_{18}$ alkyl radical;
or $R_3$ = ($C_{17}$ alkyl)amidopropyl radical and
$R_4$ = ($C_{14}$ alkyl) acetate radical, and

b) when X is $CH_3SO_4^-$:
b) when X is $CH_3SO_4^-$:

- $R_2$ represents an (alkyl and/or alkenyl)amidoethyl radical in which the alkyl and/or alkenyl radical is a $C_{13}$-$C_{21}$ radical and is derived from tallow fatty acids;
- $R_3$ and $R_4$ together with the nitrogen form a 4,5-dihydroimidazole ring substituted in position 2; and/or

2) at least one quaternised protein consisting of a chemically modified polypeptide bearing, at the end of the chain or grafted onto the latter, at least one quaternary ammonium group which contains at least one $C_1$-$C_{18}$ alkyl group, the polypeptide being selected from animal protein hydrolysates, the surface-active agent(s) representing from 0.05 to 10 % by weight relative to the total weight of the composition, the quaternised protein (s) representing from 0.05 to 3 % by weight relative to the total weight of the composition and the nonionic amphiphilic lipid(s) representing from 0.1 to 20 % by weight relative to the total weight of the composition.

**2.** Composition according to Claim 1, **characterised in that** the vesicles have an average diameter of between 0.01 and 5 µm, and preferably between 0.1 and 0.35 µm.

**3.** Composition according to either of Claims 1 and 2, **characterised in that** the nonionic amphiphilic lipid(s) is/are selected from the group consisting of linear or branched ethers and esters of polyglycerol having the respective formulae (II) and (III):

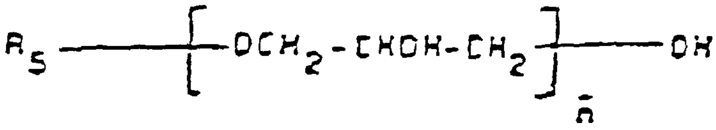

(II)

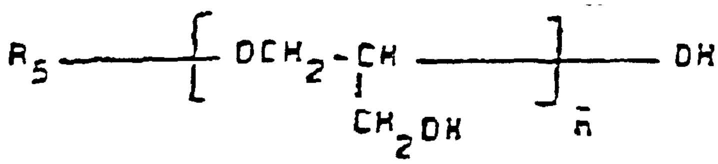

(III)

in which formulae $\bar{n}$ is an average statistical value of between 2 and 6 and $R_5$ is:

1) either an aliphatic chain $R_6$ or a residue $R'_6CO$, $R_6$ being a linear or branched $C_{12}$-$C_{18}$ aliphatic radical and $R'_6$ being a linear or branched $C_{11}$-$C_{17}$ aliphatic radical;
2) or a radical

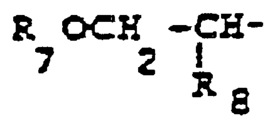

where $R_7$ and $R_8$ are radicals $R_6$ or $R'_6CO$, identical or different, $R_6$ and $R'_6$ having the meanings defined above.

**4.** Composition according to one of Claims 1 to 3, **characterised in that** the nonionic amphiphilic lipid(s) which constitute(s) the hydrated lamellar lipid phase is/are combined with at least one stabilising agent selected from the group consisting of sterols, mono- or disodium salts of acylglutamates, the acyl radical being a $C_{14}$-$C_{22}$ radical, and phosphoric esters of $C_{12}$-$C_{16}$ fatty alcohols.

**5.** Composition according to one of Claims 1 to 4, **characterised in that** the nonionic amphiphilic lipid(s) which constitute(s) the hydrated lamellar lipid phase is/are combined with at least one anionic stabilising agent in an amount at most equal to 12 % by weight and/or with at least one sterol in an amount at most equal to 100 % by weight, the proportion being calculated, in both cases, relative to the weight of the said nonionic amphiphilic lipid(s).

**6.** Composition according to Claims 3 and 4 taken in combination, in which the nonionic amphiphilic lipid(s) which constitute(s) the hydrated lamellar lipid phase comprise(s) an ether or ester of polyglycerol of formula (II) or (III) in which $R_5$ is an aliphatic chain $R_6$ or $R'_6CO$, $R_6$ and $R'_6$ having the meanings stated in Claim 3, **characterised in that** the said nonionic lipid(s) is/are combined with both a sterol and an anionic stabilising agent.

**7.** Composition according to one of Claims 1 to 6, **characterised in that** the hydrated lamellar lipid phase contains water in the presence or absence of a cosmetically or pharmaceutically active agent.

**8.** Composition according to Claim 7, in which the hydrated lamellar lipid phase contains an active agent, **characterised in that** the active agent is selected from the group consisting of agents combating hair loss or promoting hair regrowth, retinoids and related compounds, anti-inflammatory agents, antifungal agents, anti-seborrhoeic agents and sunscreen agents.

**9.** Composition according to one of Claims 1 to 8, **characterised in that** the cationic surface-active agent contained in the continuous aqueous phase is selected from the group consisting of:

a) tetraalkylammonium halides;

b) stearamidopropyldimethyl (myristyl acetate)-ammonium chloride having the formula:

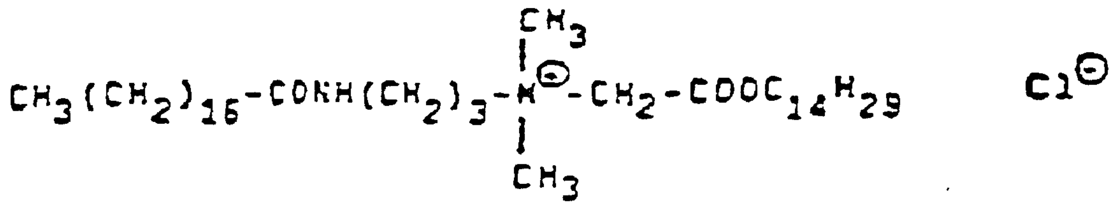

c) a quaternary ammonium salt having the formula:

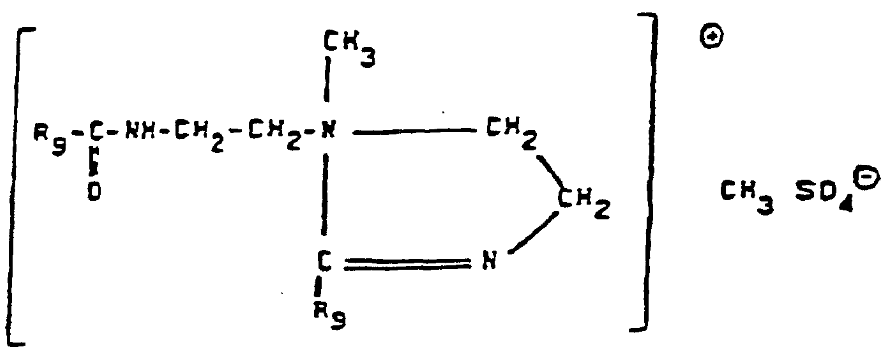

in which $R_9$ represents a mixture of $C_{13}$-$C_{21}$ alkenyl and/or alkyl radicals which is derived from tallow fatty acids.

**10.** Composition according to one of Claims 1 to 9, **characterised in that** the quaternised protein is selected from the group consisting of:

a) animal protein hydrolysates bearing on the polypeptide chain quaternary ammonium groups containing at least one $C_1$-$C_{18}$ alkyl radical;
b) animal protein hydrolysates bearing trimethylbenzylammonium groups;
c) collagen hydrolysates bearing triethylammonium groups;
d) collagen hydrolysates bearing trimethylammonium and trimethylstearylammonium groups;
e) a quaternised protein resulting from the condensation of cocamidopropyldimethylamine with a hydrolysed animal protein.

**11.** Composition according to one of Claims 1 to 10, **characterised in that** it contains both at least one cationic surface-active agent and at least one quaternised protein.

**12.** Composition according to Claim 11, **characterised in that** it contains, on the one hand behenyltrimethylammonium chloride, and on the other hand an animal protein hydrolysate bearing on the polypeptide chain quaternary ammonium groups containing at least one $C_1$-$C_{18}$ alkyl chain, the polypeptide chain having an average molecular weight of about 12,000.

**13.** Composition according to one of Claims 1 to 12, **characterised in that** the continuous aqueous phase contains at least one additive selected from the group consisting of preservatives, stabilising agents, colourings, humectants, emollients, fragrances and thickening agents.

**14.** Composition according to one of Claims 1 to 13, containing at least one cationic surface-active agent, **characterised in that** the said cationic surface-active agent represents from 0.1 to 6 % of the total weight of the composition.

**15.** Composition according to one of Claims 1 to 14, containing at least one quaternised protein, **characterised in**

**that** the said quaternised protein represents from 0.05 to 0.5 % of the total weight of the composition.

**16.** Composition according to one of Claims 1 to 15, **characterised in that** the nonionic amphiphilic lipid(s) represent(s) from 1 to 10 % by weight, and preferably from 3 to 10 % by weight, relative to the total weight of the composition.

**17.** Process for the treatment of the hair and scalp, **characterised in that** 20 to 40 g of a composition according to one of Claims 1 to 16 are applied on the head, **in that** the composition is left in contact for 1 to 15 min and **in that** the head is then rinsed with water.

**18.** Process for the treatment of the hair and scalp, **characterised in that** 5 to 10 g of a composition according to one of Claims 1 to 16 are applied on the head.